# EUROPEAN PATENT SPECIFICATION

(11) **EP 4 743 065 B1**
(45) Date of publication and mention of the grant of the patent: **18.11.2026**
(21) Application number: 25770513.7
(22) Date of filing: 23.09.2025
(51) Int. Cl.: A61K 9/20, A61K 47/26, A61K 47/38

(54) **NOVEL TABLETTING EXCIPIENT AND METHOD OF MANUFACTURE**
NEUER TABLETTIERHILFSSTOFF UND VERFAHREN ZUR HERSTELLUNG
NOUVEL EXCIPIENT POUR COMPRIMÉS ET SON PROCÉDÉ DE FABRICATION

(30) Priority: 24.09.2024 EP 24202362; 27.05.2025 EP 25179177
(43) Date of publication of application: 20.05.2026
(73) Proprietor: MEGGLE Group GmbH, 83512 Wasserburg am Inn (DE)
(72) Inventor: LEISTER, Ricarda Christina, 83135 Pfaffenhofen (DE); FICHTNER, Vera, 83512 Reitmehring (DE); ZIMPEL, Andreas, 83543 Rott am Inn (DE); FRIEDRICH, Jan, 83549 Eiselfing (DE)
(74) Representative: Grünecker Patent- und Rechtsanwälte PartG mbB
(86) International application number: PCT/EP2025/077234
(87) International publication number: WO 2026/068479

(56) References cited:
- CN-A- 114 796 507
- US-A1- 2013 177 649
- PHARMATRANS SANAQ AG SUPPLIER: "DICOM SANAQ ML 011", 22 October 2023 (2023-10-22), pages 1 - 1, XP093249500, Retrieved from the Internet <URL:https://www.pharmaexcipients.com/wp-content/uploads/2023/01/PTS_Specifications_CoProcessed_ML_011_DiCom-SANAQ -ML-011.pdf>
- GOHEL M C ET AL: "An investigation of the direct-compression characteristics of coprocessed lactose-microcrystalline cellulose using statistical design", PHARMACEUTICAL TECHNOLOGY, ADVANSTAR COMMUNICATIONS, EUGENE, OR, US, vol. 23, no. 11, 1 November 1999 (1999-11-01), pages 54 - 62, XP009125297, ISSN: 0147-8087

## Description

### TECHNICAL FIELD

The present invention relates to the field of pharmaceutical excipients and methods for their manufacture. In particular, the present invention relates to a novel co-processed excipient comprising 10-40 wt% lactose and 60-90 wt% microcrystalline cellulose (MCC). The present invention further relates to a novel method of preparing the novel excipient comprising preparing an aqueous suspension of MCC and lactose, wherein the dry mass of the suspension comprises 60-90 wt% MCC and 10-40 wt% lactose; heating the suspension to a temperature of at least about 50°C to form a heated slurry; and spray-drying the heated slurry, thereby obtaining a co-processed excipient comprising 10-40 wt% lactose and 60-90 wt% MCC. The present invention further relates to uses of the novel co-processed excipients for direct tableting as well as tablets prepared from the novel co-processed excipients.

### BACKGROUND ART

Various methods for producing tablets are known. Direct tableting is the simplest and most economical way to produce tablets, as tableting can take place directly after mixing all the necessary ingredients and components without the need for further process steps such as granulation of active components and tableting aids. Excipients with good tableting properties may be required for dosing during capsule filling of difficult active ingredients, such as, e.g., plant extracts.

A tableting aid, in particular a tableting excipient or pharmaceutical excipient, for direct tableting must fulfil a number of properties. For example, good compressibility with low compression pressure is required, as is a good flowability. The tablets produced from this material must have a high breaking strength and abrasion resistance, but at the same time must also dissolve quickly on contact with gastric juice.

By simply physically mixing commercially available individual tableting excipients, the necessary properties of directly compressible tablet formulations described above are not completely fulfilled in many, if not most cases.

Lactose and cellulose are the most commonly used tableting excipients. Both components are used in many tablet formulations, as the two substances have complementary behavior and complement each other well. Cellulose shows a plastic deformation behavior and lactose, in contrast, a brittle deformation behavior.

US 4693750 describes a granulate of starch and lactose as an aid for direct tableting. EP 3200771 B1 describes a direct tableting aid consisting of at least one lactose, one starch and one cellulose component. The combination with starch is considered to result in rapid disintegration but lower breaking strength. Pharmatrans Sanaq Ag Supplier: "Dicom Sanaq ML 011", 2023, is a product specification referring to a co-processed excipient for direct tableting consisting of 40 wt% lactose monohydrate and 60 wt% microcrystalline cellulose.

It is constantly desired to provide co-processed direct tableting excipients for producing tablets, which exhibit a high breaking strength and which largely fulfil the other required properties as discussed above. This applies in particular in case of higher loads of active ingredients or in case of particularly poorly compressible active ingredients, such as paracetamol. For such formulations, wet granulation is still commonly used.

The present invention addresses the needs in the art and provides a novel excipient that facilitates the production of tablets by means of direct tableting. The novel excipients are considered to be particularly useful for direct tableting even in case of high active ingredient load and poor compressibility.

### SUMMARY OF THE INVENTION

The present invention relates to a novel co-processed excipient for the production of tablets by means of direct tableting (tableting excipient), as well as a method for its production. The novel tableting excipient provided by the present invention shows advantageous tableting properties, which are due to the specific combination of the co-processed components and the method of manufacture of the excipient. The present invention is defined by the appended claims.

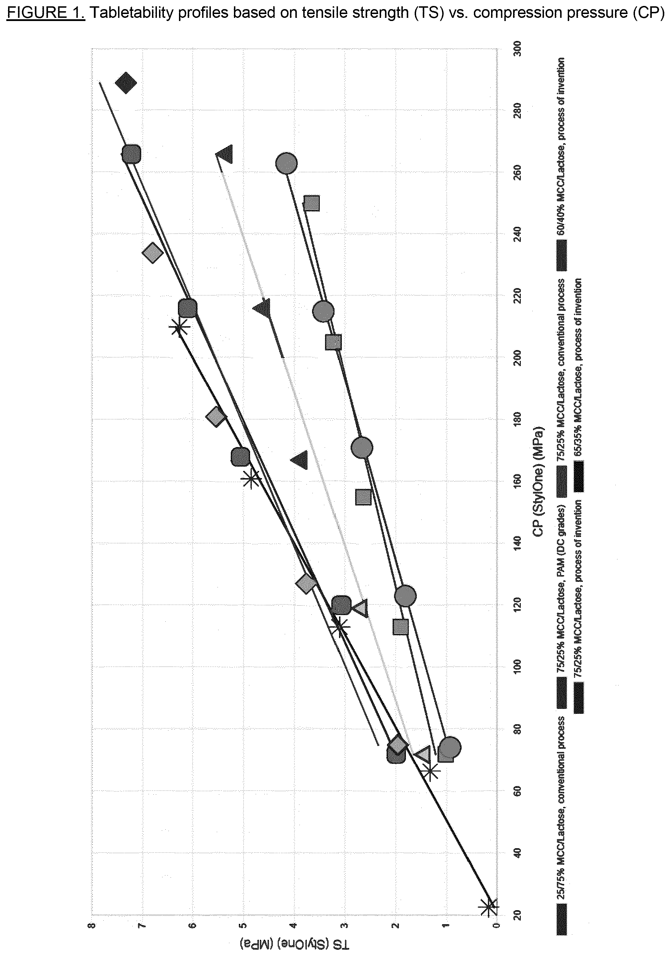

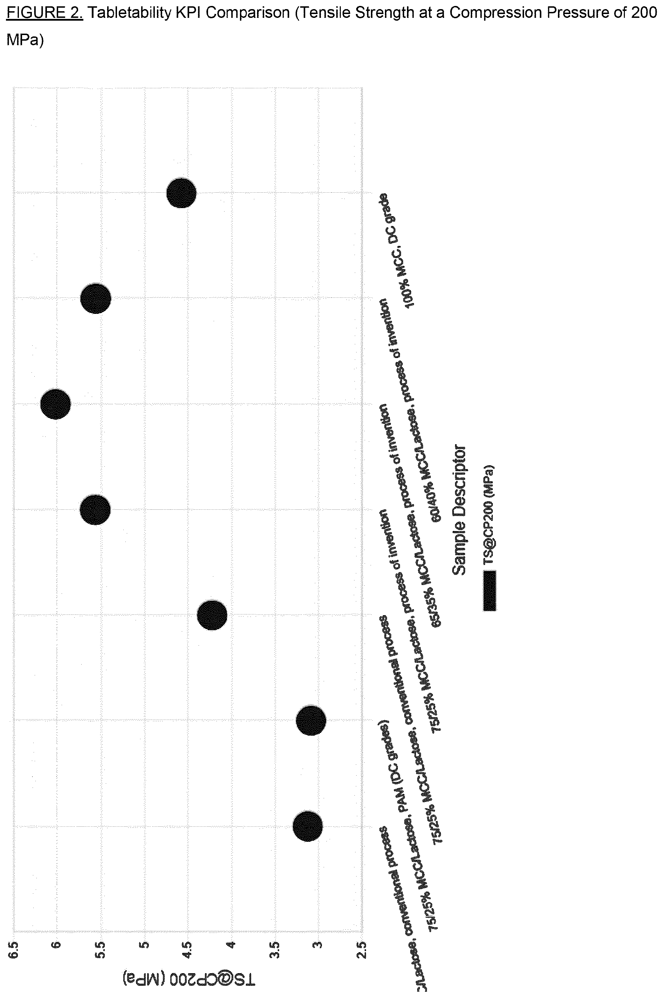

Aspects and embodiments of the invention include:
[1] A co-processed excipient comprising 10-40 wt% lactose and 60-90 wt% microcrystalline cellulose (MCC), wherein the co-processed excipient is generated by a method comprising:
   (i) preparing an aqueous suspension of MCC;
   (ii) adding lactose to the aqueous suspension of (i), wherein the dry mass of the aqueous suspension thus obtained comprises 60-90 wt% MCC and 10-40 wt% lactose;
   (iii) heating the aqueous suspension of (ii) to a temperature of at least about 50°C to form a heated slurry;
   (iv) spray-drying the heated slurry of (iii);
   (v) obtaining a co-processed excipient comprising 10-40 wt% lactose and 60-90 wt% MCC.
   The present invention can also be performed with steps (i) and (ii) carried out in reverse order: (i) preparing an aqueous suspension of lactose; (ii) adding MCC to the aqueous suspension of (i) (wherein the dry mass of the aqueous suspension thus obtained comprises 60-90 wt% MCC and 10-40 wt% lactose).
[2] The co-processed excipient of [1], comprising 25-40 wt% lactose and 60-75 wt% MCC, or comprising 20-30 wt% lactose and 70-80 wt% MCC, or comprising 30-40 wt% lactose and 60-70 wt% MCC.
[3] The co-processed excipient of [1] or [2], comprising about 60 wt% MCC, more specifically comprising about 60 wt% MCC and about 40 wt% lactose. In various preferred embodiments, the co-processed excipient comprises about 65 wt% MCC, more specifically about 65 wt% MCC and about 35 wt% lactose. In various other preferred embodiments, the co-processed excipient comprises about 75 wt% MCC, more specifically about 75 wt% MCC and about 25 wt% lactose.
   Accordingly, a preferred range is at least about 60% wt% MCC to at least about 75 wt% MCC, and/or at least about 40 wt% lactose to at least about 25 wt% lactose.
[4] The co-processed excipient of any one of [1] to [3], wherein the median diameter of the particles of the excipient is in the range from about 60 µm to about 150 µm.
[5] A tablet prepared from the co-processed excipient according to any one of [1] to [4], preferably by direct compression.
[6] The tablet of [5], which has a higher tensile strength, or a higher tabletability (tensile strength vs. compression pressure), as compared to a tablet prepared from a physical mixture of the same concentration of lactose and MCC. The terms "physical mixture" and "physical admixture" may be used interchangeably herein. As further described herein, the tablet of [6] has a higher tensile strength, or a higher tabletability (tensile strength vs. compression pressure), as compared to a direct compression (DC) product of the individual components prepared by spray drying of lactose, in particular (alpha) lactose monohydrate, or MCC, respectively. A known spray-dried alpha lactose monohydrate DC product is marketed as FlowLac^{®}. As can be derived from Figure 1, a tablet prepared from a co-processed excipient of the present invention, in particular from a co-processed excipient comprising 25-40 wt% lactose and 60-75 wt% microcrystalline cellulose (MCC), shows advantages properties in tensile strength, in particular a tensile strength of at least 5.0 (MPa), or greater than or ≥ 5.0 (MPa), at a compression pressure of 200 MPa. As further evidenced by Figure 2, the tensile strength at a compression pressure of 200 MPa can be in the range between 5.0 MPa and 6.5 MPa. In various embodiments, the tensile strength at a compression pressure of 200 MPa is in the range of (about) 5.4 MPa and (about) 6.1 MPa, more specifically in the range of (about) 5.5 MPa and (about) 6.0 MPa. As described elsewhere herein, in particularly preferred embodiments, a co-processed excipient comprises 30-40 wt% lactose and 60-70 wt% MCC.
   While the use of the term "compression pressure" follows the United States Pharmacopeia (United States Pharmacopeia (2023). *General Chapter, 〈1062〉 Tablet Compression Characterization.* USP-NF. Rockville, MD: United States Pharmacopeia. DOI: https://doi.org/10.31003/USPNF_M99395_02_01. Doc ID: GUID-A53EEC6D-BF32-464C-88BC-5303A5628C2C_2_en-US), the terms "compression pressure" and "compaction pressure" may be used interchangeably in the art. Accordingly, the terms "compression pressure" and "compaction pressure" may also be used interchangeably in the present invention.
[7] The tablet of [5], which has a higher tensile strength, or a higher tabletability (tensile strength vs. compression pressure), as compared to a tablet prepared from a co-processed excipient comprising 75 wt% lactose, in particular 75 wt% (alpha) lactose monohydrate and 25 wt% MCC.
[8] A method of preparing a co-processed excipient comprising 10-40 wt% lactose and 60-90 wt% microcrystalline cellulose (MCC), wherein the method comprises:
   (i) preparing an aqueous suspension of MCC;
   (ii) adding lactose to the aqueous suspension of (i), wherein the dry mass of the aqueous suspension thus obtained comprises 60-90 wt% MCC and 10-40 wt% lactose;
   (iii) heating the aqueous suspension of (ii) to a temperature of at least about 50°C to form a heated slurry;
   (iv) spray-drying the heated slurry of (iii);
   (v) obtaining a co-processed excipient comprising 10-40 wt% lactose and 60-90 wt% MCC.
   In accordance with [1], steps (i) and (ii) of the method can be carried out in reverse order: (i) preparing an aqueous suspension of lactose; (ii) adding MCC to the aqueous suspension of (i) (wherein the dry mass of the aqueous suspension thus obtained comprises 60-90 wt% MCC and 10-40 wt% lactose).
[9] The method of [8], wherein:
   a) the MCC of step (i) has a (median) particle size of <100 µm; and/or
   b) step (iii) comprises heating the aqueous suspension to a temperature in the range from about 50°C to about 100°C.
[10] The method of [8] or [9], wherein the spray-drying of step (iv) is performed at a temperature in the range of about 140°C to about 225°C.
[11] Use of the co-processed excipient according to any one of [1] to [4] in a process of preparing a tablet by direct compression.
[12] Use of an aqueous suspension for preparing a co-processed excipient comprising 10-40 wt% lactose and 60-90 wt% microcrystalline cellulose (MCC), wherein the dry mass of the suspension comprises 10-40 wt% lactose and 60-90 wt% MCC, and wherein the lactose is dissolved in the liquid phase of an aqueous suspension of MCC, and wherein the co-processed excipient is generated by a method comprising:
   (i) heating the aqueous suspension to a temperature of at least about 50°C to form a heated slurry;
   (ii) spray-drying the heated slurry of (i);
   (iii) obtaining a co-processed excipient comprising 10-40 wt% lactose and 60-90 wt% MCC. The step of heating the aqueous suspension is preferably performed at a temperature in the range from about 50°C to about 100°C.
[13] A method of preparing a tablet comprising direct compression of a (pharmaceutical) blend of an active pharmaceutical ingredient and the co-processed excipient of any one of [1] to [4].

### BRIEF DESCRIPTION OF THE DRAWINGS

**Figure 1****:** shows tabletability profiles based on tensile strength (TS) vs. compression pressure (CP). Circles show the tabletability profile of a composition of 25/75 wt% MCC/Lactose prepared from a co-spray dried mixture of 25 wt% MCC and 75 wt% lactose ("conventional" spray-drying process; MicroceLac^{®} 100). Squares show the tabletability profile of a composition of 75/25 wt% MCC/Lactose prepared from a physical admixture (PAM) of 75 wt% MCC and 25 wt% lactose. Triangles show the tabletability profile of a composition of 75/25 wt% MCC/Lactose prepared from a co-spray dried mixture of 75 wt% MCC and 25 wt% lactose ("conventional" spray-drying process). Diamonds show the tabletability profile of a composition of 60/40 wt% MCC/Lactose prepared according to the spray drying process of the invention using 60 wt% MCC and 40 wt% lactose. Squares with round corners show the tabletability profile of a composition of 75/25 wt% MCC/Lactose prepared according to the spray drying process of the invention using 75 wt% MCC and 25 wt% lactose. Stars show the tabletability profile of a composition of 65/35 wt% MCC/Lactose prepared according to the spray drying process of the invention using 65 wt% MCC and 35 wt% lactose. TS = tensile strength; CP = compression pressure; Lactose = (alpha) lactose monohydrate.
**Figure 2****:** shows a tabletability KPI comparison based on tensile strength at a compression pressure of 200 MPa. The sample description follows the sample description of Figure 1 (MicroceLac^{®} 100 shown at the very left). Furthermore, "100% MCC grade" refers to a direct compression (DC) product of 100 wt % MCC prepared by spray drying. TS = tensile strength; CP = compression pressure; Lactose = (alpha) lactose monohydrate. The tensile strength at a compaction pressure of 200 MPa ("TS@CP200") is calculated using linear interpolation from the tabletability profile (tensile strength vs compaction pressure).

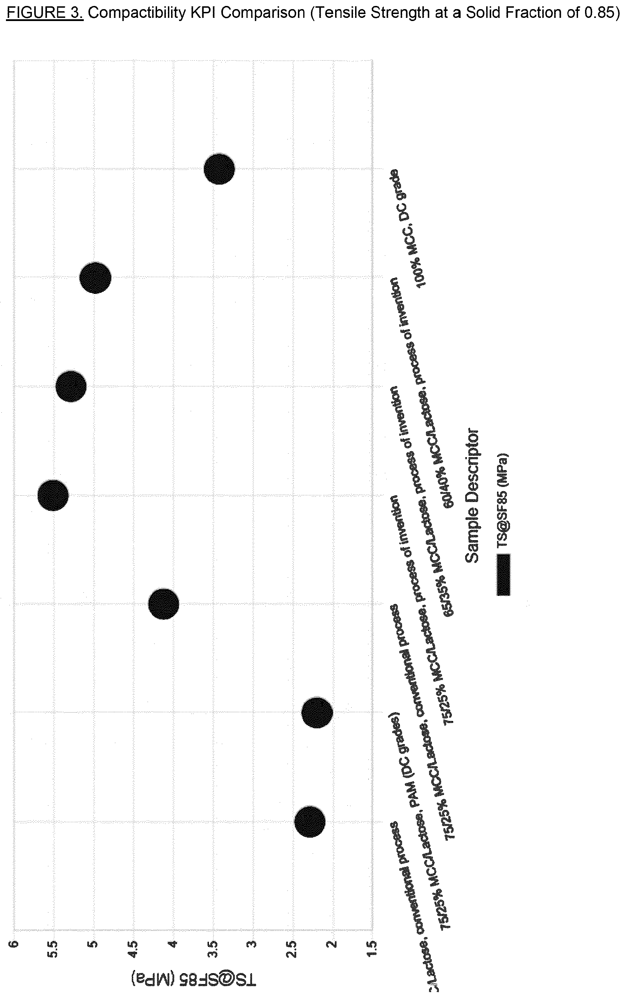

**Figure 3****:** shows a compactibility KPI comparison based on tensile strength at a solid fraction of 0.85 (remaining porosity 15%). The relationship between tensile strength and solid fraction (or porosity) is termed "compactibility". "100% MCC grade" refers to a direct compression (DC) product of 100 wt % MCC prepared by spray drying. Otherwise, the sample description follows the sample description of Figure 2 (MicroceLac^{®} 100 shown at the very left). TS = tensile strength; CP = compression pressure; Lactose = (alpha) lactose monohydrate. The tensile strength at a solid fraction of 0.85 ("TS@SF85") is calculated using interpolation based on exponential fit from the compactibility profile (tensile strength vs compaction pressure).

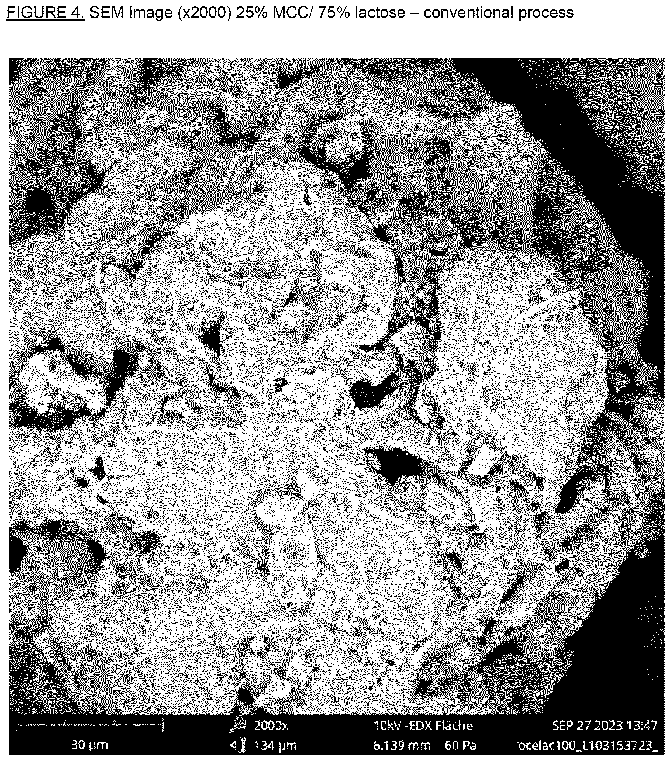

**Figure 4****:** shows an SEM image (x2000) of a composition of 25/75 wt% MCC/Lactose prepared from a co-spray dried mixture of MCC and lactose ("conventional" spray-drying process; MicroceLac^{®} 100).

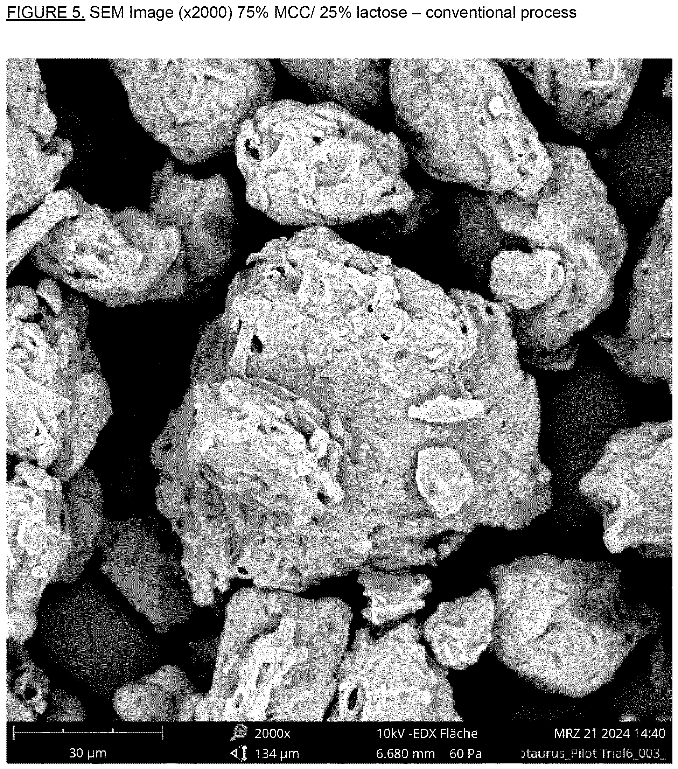

**Figure 5****:** shows an SEM image (x2000) of a composition of 75/25 wt% MCC/Lactose prepared from a co-spray dried mixture of MCC and lactose ("conventional" spray-drying process).

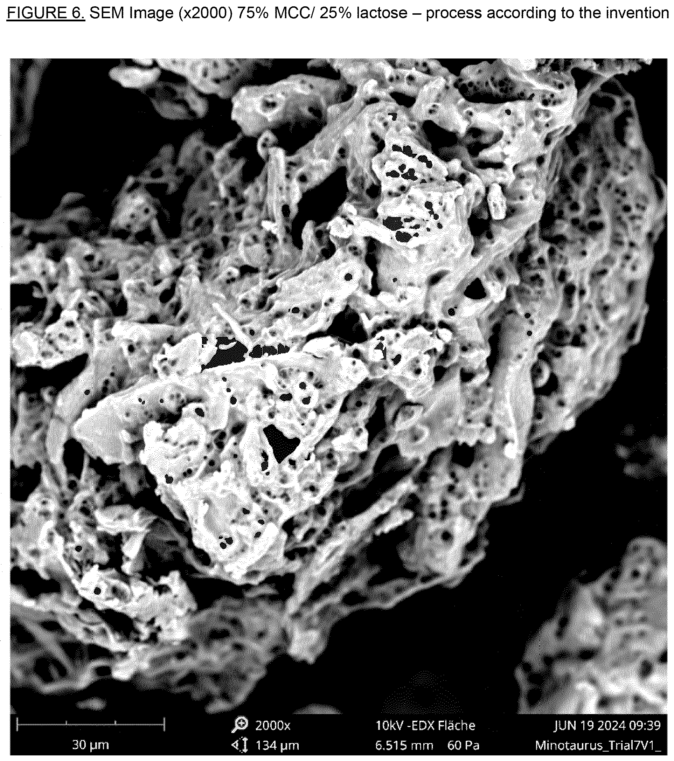

**Figure 6****:** shows an SEM image (x2000) of a composition of 75/25 wt% MCC/Lactose prepared according to the spray drying process of the invention.

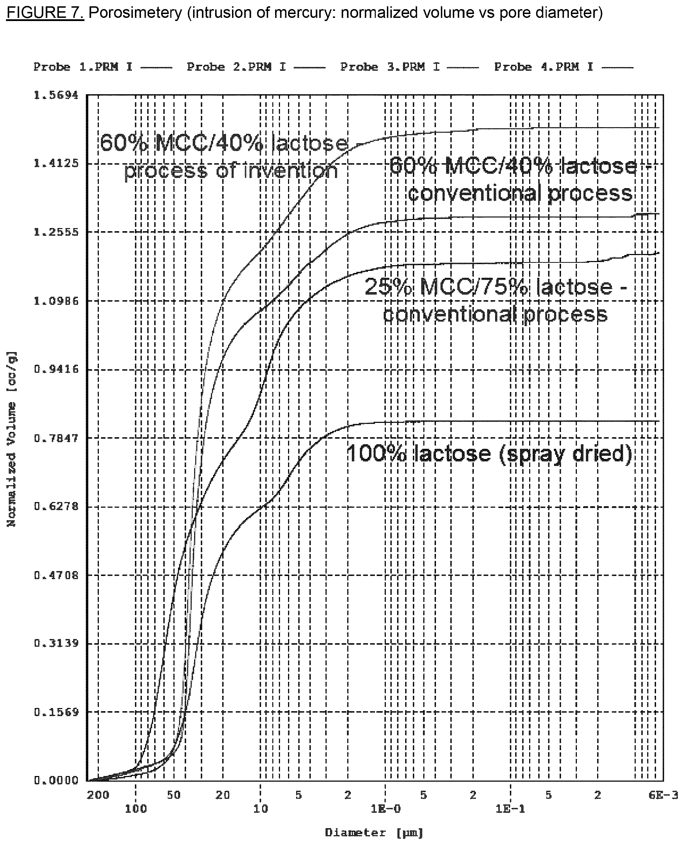

**Figure 7****:** shows a pore size measurement based on mercury porosimetry (intrusion of mercury: normalized volume vs pore diameter) applying Quantochrome Poremaster ISO 15901-1: bulk sample volume 1 cm³, measurement range approx. 1,000 µm-0.0036 µm pore diameter. The granular density includes contributions to particle volume from open, small pores. "100% lactose" refers to a direct compression (DC) product of 100 wt % (alpha) lactose monohydrate (FlowLac^{®}). "60% MCC/40% lactose - conventional process" refers to a composition of 60/40 wt% MCC/Lactose prepared from a co-spray dried mixture of a composition of 60 wt% MCC and 40 wt% lactose ("conventional" spray-drying process). Otherwise, the sample description follows the sample description of Figure 2.

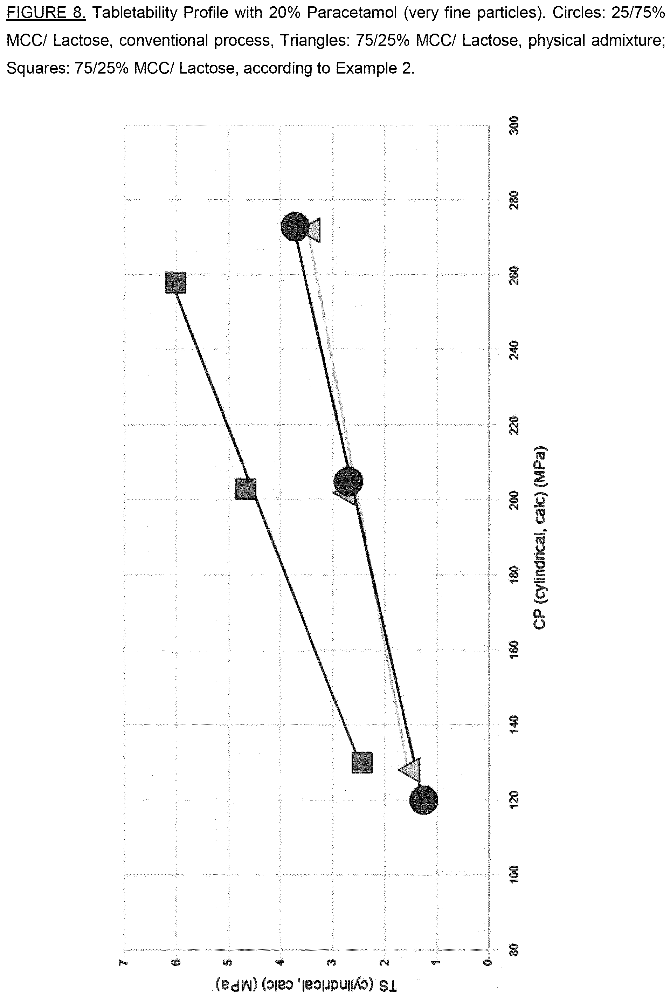

**Figure 8****:** shows a tabletability profile with 20% Paracetamol (very fine) based on tensile strength (TS) vs. compression pressure (CP). Circles show the tabletability profile of a composition of 25/75 wt% MCC/Lactose prepared from a co-spray dried mixture of 25 wt% MCC and 75 wt% lactose ("conventional" spray-drying process). Triangles show the tabletability profile of a composition of 75/25 wt% MCC/Lactose prepared from a physical admixture (PAM) of 75 wt% MCC and 25 wt% lactose. Squares show the tabletability profile of a composition of 75/25 wt% MCC/Lactose prepared according to Example 2. TS = tensile strength; CP = compression pressure; Lactose = (alpha) lactose monohydrate.

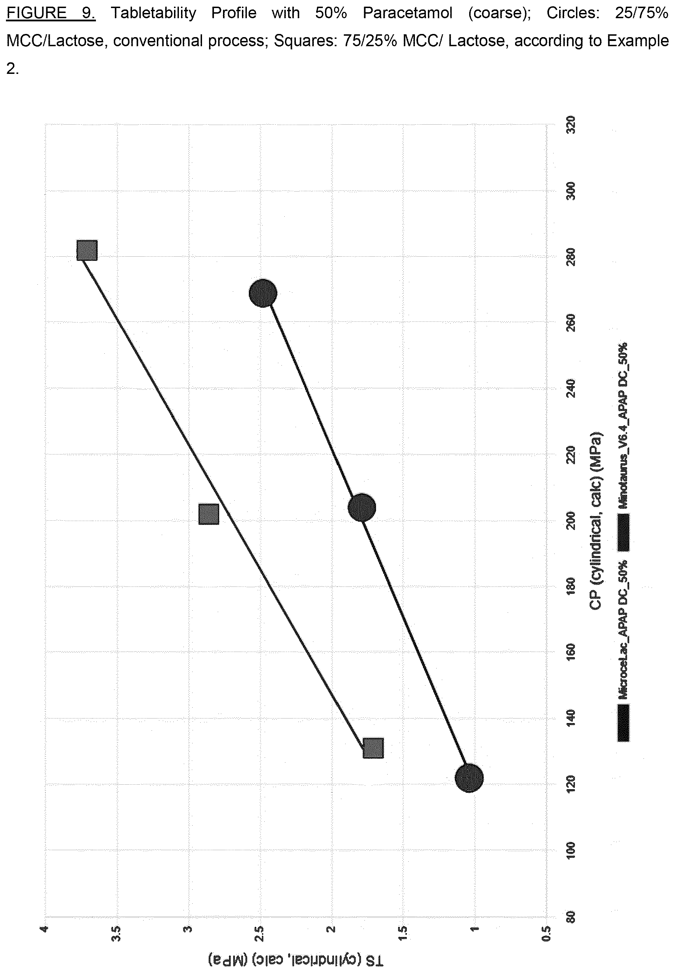

**Figure 9****:** shows a tabletability profile with 50% Paracetamol (coarse) based on tensile strength (TS) vs. compression pressure (CP). Circles show the tabletability profile of a composition of 25/75 wt% MCC/Lactose prepared from a co-spray dried mixture of 25 wt% MCC and 75 wt% lactose ("conventional" spray-drying process). Squares show the tabletability profile of a composition of 75/25 wt% MCC/Lactose prepared according to Example 2. TS = tensile strength; CP = compression pressure; Lactose = (alpha) lactose monohydrate.

In all Figures 1 to 7, "%" means "wt %" based on total dry weight of the (co-processed) excipient or composition. In Figures 8 and 9, "%" means "wt %" based on total weight of the tablet.

### DEFINITIONS

As used herein, the use of the terms "a" or "an" connotes "one or more" or "at least one", and the subsequent use of the definite articles "the" or "said" in an embodiment or a claim referring back to the same term does not change the general plural rule, but invokes that non-singular meaning, unless otherwise specified.

As used herein, the terms "active ingredient" and "active pharmaceutical ingredient" ("API") refer to any ingredient that provides a biologically active effect or other direct effect in the diagnosis, cure, mitigation, therapy (including treatment and prevention) of a disease or (medical) condition, or that affects the structure or any function of the body of humans or animals.

As described herein, the term "active substance" may be used for natural products. A natural product is a compound or substance produced by a living organism, i.e., a compound or substance found in nature.

The terms "co-processed excipient" or "pharmaceutical excipient" or "co-processed pharmaceutical excipient" may be used interchangeably herein.

Co-processed excipients are typically used in the pharmaceutical industry. The term pharmaceutical (co-processed) excipient is sometimes used to describe such a co-processed excipient for use in pharmaceutical applications, in particular for tableting. As described herein, and as appreciated by a person skilled in the art, a pharmaceutical excipient is basically everything other than the active pharmaceutical ingredient.

As described herein, the terms "pharmaceutical composition" and "composition for therapeutic treatment" may be used interchangeably herein.

The terms "comprising" or "comprise(s)" include, where applicable or necessary, the indication "consisting of" or "consist(s) of", respectively.

### DETAILED DESCRIPTION OF THE INVENTION

The present invention provides a novel co-processed excipient comprising microcrystalline cellulose (MCC) and lactose in a (mass) ratio (in the range) of about 60:40 to about 90:10. Specifically, or alternatively, the present invention provides a novel co-processed excipient comprising about 60-90 wt% microcrystalline cellulose (MCC) and about 10-40 wt% lactose.

The present invention provides a novel method of preparing the novel co-processed excipient, wherein the method is generally characterized by process steps including (i) preparing an aqueous suspension of microcrystalline cellulose (MCC) and lactose, wherein the dry mass of the aqueous suspension comprises MCC and lactose in a (mass) ratio (in the range) of about 60:40 to about 90:10 (or comprises about 60-90 wt% MCC and about 10-40% lactose); (ii) heating the aqueous suspension of (i) to a temperature of at least about 50°C to form a heated slurry; (iii) spray-drying the heated slurry of (ii) and thereby obtaining a co-processed excipient comprising MCC and lactose in a (mass) ratio (in the range) of about 60:40 to about 90:10 (or comprising about 60-90 wt% MCC and about 10-40 wt% lactose).

The step of preparing the aqueous suspension of MCC and lactose is preferably performed by first preparing an aqueous suspension of MCC, wherein the dry mass of the aqueous suspension comprises about 60-90 wt% MCC, followed by adding lactose to the aqueous suspension of MCC, wherein the dry mass of the aqueous suspension thus obtained comprises about 60-90 wt% MCC and about 10-40 wt% lactose. Accordingly, the present invention (further) provides a novel method of preparing the novel co-processed excipient, wherein the method is characterized by process steps including (i) preparing an aqueous suspension of MCC, wherein the dry mass of the aqueous suspension comprises 60-90 wt% MCC; (ii) adding 10-40 wt% lactose to the aqueous suspension of (i), wherein the dry mass of the aqueous suspension thus obtained comprises about 60-90 wt% MCC and about 10-40 wt% lactose; (iii) heating the aqueous suspension of (ii) to a temperature of at least 50°C to form a heated slurry; and (iv) spray-drying the heated slurry of (iii) and thereby obtaining a co-processed excipient comprising about 60-90 wt% MCC and about 10-40 wt% lactose (or comprising MCC and lactose in a (mass) ratio (in the range) of about 60:40 to about 90:10).

The "obtaining" of the novel co-processed excipient may be formulated as a separate step, as depicted in claim 1, *i.e.,* as a step (v) describing (thereby) obtaining a co-processed excipient comprising about 60-90 wt% MCC and about 10-40 wt% lactose (or comprising MCC and lactose in a (mass) ratio (in the range) of about 60:40 to about 90:10).

As described herein, the step(s) of preparing the aqueous suspension comprising MCC and lactose, in particular the steps of preparing an aqueous suspension of MCC, wherein the dry mass of the aqueous suspension comprises about 60-90 wt% MCC, and/or adding lactose to the aqueous suspension of MCC, wherein the dry mass of the aqueous suspension thus obtained comprises about 60-90 wt% MCC and about 10-40 wt% lactose, may already involve heating or warming of the respective aqueous suspension(s). Such heating or warming, if applied at all, may involve a heating of the respective aqueous suspension(s) comprising MCC and lactose to a temperature of least about 50°C during the step of adding the lactose to the aqueous suspension of MCC. In such a situation, the heated slurry having a temperature of about 50°C is formed at the end of adding the lactose to the aqueous suspension of MCC. The separate heating step can then be omitted and the heated slurry can then be (directly) spray-dried, as described elsewhere herein.

Accordingly, the present invention encompasses a novel method of preparing the novel co-processed excipient, which is characterized by process steps including (i) preparing a heated slurry of MCC and lactose, wherein the heated slurry is prepared by heating an aqueous suspension of MCC and lactose to a temperature of at least about 50°C, wherein the dry mass of the aqueous suspension comprises MCC and lactose in a (mass) ratio (in the range) of about 60:40 to about 90:10 (or comprises about 60-90 wt% MCC and about 10-40% lactose); (ii) spray-drying the heated slurry of (i) and thereby obtaining a co-processed excipient comprising MCC and lactose in a (mass) ratio (in the range) of about 60:40 to about 90:10 (or comprising about 60-90 wt% MCC and about 10-40% lactose). Again, the "obtaining" of the novel co-processed excipient may be formulated as a separate step, *i.e.,* in this case a step (iii) describing (thereby) obtaining a co-processed excipient comprising MCC and lactose in a (mass) ratio (in the range) of about 60:40 to about 90:10 (or comprising about 60-90 wt% MCC and about 10-40% lactose). Further, the step of (i) preparing the heated slurry of MCC and lactose may comprise first preparing an aqueous suspension of MCC, wherein the dry mass of the aqueous suspension comprises about 60-90 wt% MCC, followed by adding lactose to the aqueous suspension of MCC, wherein the dry mass of the aqueous suspension thus obtained comprises about 60-90 wt% MCC and about 10-40 wt% lactose, and heating the suspension to a temperature of at least about 50°C during (or while) adding the lactose to the aqueous suspension of MCC.

The present invention is the first to describe, *inter alia,* that the heated slurry is spray-dried, *i.e.,* the spray-drying is carried out with the heated slurry without waiting for the slurry to cool down, which is the case in the "conventional process" referred to herein (e.g., in the Examples and the Figures). In particular, in the "conventional process" referred to herein, the lactose is typically added to the cellulose suspension only after cooling down of the cellulose suspension.

In contrast, as described herein, in the "process of the invention" (or "process of invention") referred to herein, the aqueous suspension comprising MCC and lactose is heated to a temperature of at least about 50°C to form a heated slurry (or heated liquid feed) for spray drying.

Accordingly, in the present invention, spray-drying is performed using a slurry for spray drying that is heated to a temperature of at least about 50°C. More specifically, the slurry (or liquid feed) for spray drying may be heated to a temperature in the range from about 50°C to about 100°C, in accordance with the embodiment described elsewhere herein, according to which the step of heating the aqueous suspension ("step (iii)" of the process steps of the method) may be a heating to a temperature in the range from about 50°C to about 100°C.

In preferred embodiments of the invention, the step of heating the aqueous suspension of MCC and lactose is a heating to a temperature in the range from about 70°C to about 85°C, more preferably from about 75°C to about 80°C.

Accordingly, in the present invention, the step of spray-drying the heated slurry is performed with a heated slurry having a temperature of at least about 50°C, more specifically having a temperature in the range from about 50°C to about 100°C. More specifically, the heated slurry fed to the spray dryer ("liquid feed") has a temperature of at least about 50°C, more specifically a temperature in the range from about 50°C to about 100°C. Preferably, the heated slurry (fed to the spray dryer) has a temperature in the range from about 70°C to about 85°C, more preferably from about 75°C to about 80°C.

As described herein, the heated slurry is fed into a spray dryer, which may be a spray drying tower (or spray tower). As further described herein, the spray dryer, which may be a spray drying tower (spray tower), can be heated to temperatures above the boiling point of water. Accordingly, in various embodiments of the present invention, the step of spray-drying the heated slurry comprises spray-drying the heated slurry using a spray drying tower.

As described herein, in various embodiments of the present invention, the step of spray-drying the heated slurry comprises spray-drying the heated slurry using a spray drying tower (spray tower), which is heated to temperatures above the boiling point of water, in particular which is heated to temperatures above the boiling point of water before the heated slurry is fed into the spray dryer. Such a heating to temperatures above the boiling point of water in particular means heating to temperatures of about 140°C to about 225°C, in accordance with the embodiment described elsewhere herein, according to which the spray-drying ("of step (iv)" of the process steps of the method) is performed at a temperature in the range of about 140°C to about 225°C.

Accordingly, the present invention encompasses the feeding of a heated slurry having a temperature of at least about 50°C to a spray dryer and (performing the) spray drying of the heated slurry at a temperature in the range of about 140°C to about 225°C.

In preferred embodiments of the present invention, the spray-drying ("of step (iv)" of the process steps of the method) is performed at a temperature in the range of about 180°C to about 220°C, more preferably in the range of about 180°C to about 210°C, even more preferably in the range of about 180°C to about 200°C. In various preferred embodiments, the spray-drying ("of step (iv)" of the process steps of the method) is performed at a temperature in the range of about 180°C to about 190°C. In various other preferred embodiments, the spray-drying ("of step (iv)" of the process steps of the method) is performed at a temperature in the range of about 190°C to about 200°C. These preferred temperatures/temperature ranges apply accordingly to the above-described heating of the spray dryer or spray drying tower (spray tower) before the heated slurry is fed into the spray dryer.

As described herein, crystalline lactose can contain amorphous forms of lactose. In the novel co-processed excipients of the present invention, the lactose may be present partly in crystalline form and partly in amorphous form.

**Figures 4 to 6** show SEM images of different MCC/Lactose compositions prepared according to a "conventional" spray-drying process (Figures 4 and 5) or prepared according to the spray drying process of the invention (Figure 6), respectively. It has surprisingly been found that MCC/Lactose compositions according to the present invention (Figure 6) exhibit advantageous properties in terms of porosity, which is beneficial for (increasing) drug load of tablets, in particular as regards high dose tablets. The advantageous properties in terms of porosity are reflected in **Figure 3****,** which shows that excipients according to the present invention provide for an improved compactibility, which is the tablet's tensile strength as a function of the solid fraction. In other words, the relationship between tensile strength and solid fraction (or porosity where porosity = 1 - solid fraction) is termed "compactibility". Tablet solid fraction, which is also referred to as relative density, is a measure of the volume of solid material in a compact and may be calculated using the equation for "Solid Fraction" shown in the Examples section herein below. At a solid fraction of 0.85 ("SF85" in Figure 3), tablets prepared from excipients of the invention yield the strongest compact. Surprisingly, the excipients of the present invention enable tablets with a high remaining porosity (0.15) at a very stable compact (porosity = 1 - solid fraction). The remaining porosity can be important for the dissolution of the API and can be considered to protect pressure sensitive APIs. Surprisingly, Figure 3 reveals that tablets prepared from the novel excipients of the present invention show a significantly improved tensile strength at the same remaining porosity (0.85 for all tablets samples tested). The behavior of the novel excipients shown in Figure 3 is consistent with the tabletability profile shown in **Figure 1** and **Figure 2****.** Accordingly, as can be derived from **Figure 3****,** a tablet prepared from a co-processed excipient of the present invention, in particular from a co-processed excipient comprising 25-40 wt% lactose and 60-75 wt% microcrystalline cellulose (MCC), shows advantages properties in tensile strength, in particular a tensile strength of at least 4.5 (MPa), or greater than or ≥ 4.5 (MPa), at a solid fraction of 0.85. As further evidenced by Figure 3, the tensile strength at a solid fraction of 0.85 can be in the range between 4.5 MPa and 6.0 MPa. In various embodiments, the tensile strength at a solid fraction of 0.85 is in the range of (about) 4.5 MPa and (about) 6.0 MPa, more specifically in the range of (about) 5.0 MPa and (about) 5.5 MPa. As described elsewhere herein, in particularly preferred embodiments, a co-processed excipient comprises 30-40 wt% lactose and 60-70 wt% MCC.

The effects shown in Figures 3 and 4 to 6 are further confirmed by **Figure 7****,** which shows a pore size measurement (based on mercury porosimetry) of different MCC/Lactose compositions prepared according to a "conventional" spray-drying process or according to the spray drying process of the present invention. In particular, Figure 7 shows an increased normalized volume for MCC/Lactose compositions of the invention (exemplified by 60% MCC/40% lactose), in particular for MCC/Lactose compositions of the invention (exemplified by 60% MCC/40% lactose) that are prepared according to the process of the present invention.

As will be appreciated by a skilled person reading the present application, a co-processed excipient comprising about 25-35 wt% lactose and about 65-75 wt% MCC can be considered of particular practicability.

The novel excipients of the present invention, and likewise tablets prepared from the novel co-processed excipients of the present invention (in particular prepared by direct compression), can be characterized by any of the functional properties shown in the Examples and in the Figures. Accordingly, in preferred embodiments of the present invention, the novel excipients of the present invention, and likewise tablets prepared from the novel co-processed excipients of the present invention (in particular prepared by direct compression), exhibit the tensile strength, or tabletability (tensile strength vs. compression pressure) properties, and/or compactibility properties (relationship between tensile strength and solid fraction (or porosity), shown in the Examples and/or the Figures. For example, as will be appreciated by a skilled person reading the Examples and the Figures, a tablet prepared from the co-expressed excipient exhibits a tensile strength of ≥ 5.0 MPa at a solid fraction of 0.85, as demonstrated in Figure 3. As will be further appreciated by a skilled person reading the Examples and the Figures, a tablet prepared from the co-expressed excipient exhibits a tensile strength of ≥ 5.0 MPa at a compaction pressure of 200 MPa.

As described herein, the tensile strength at a compaction pressure of 200 MPa ("TS@CP200") can be calculated using linear interpolation from the tabletability profile (tensile strength vs compaction pressure). As further described herein, the tensile strength at a solid fraction of 0.85 ("TS@SF85") is calculated using interpolation based on exponential fit from the compactibility profile (tensile strength vs compaction pressure).

In particular, in various embodiments, a tablet of the invention has a higher tensile strength, or a higher tabletability, as compared to a tablet prepared from a physical mixture (or physical admixture, "PAM") of the same concentration of lactose and MCC. See "squares" (75/25% MCC/Lactose, PAM (DC grades) in Figure 1.

In various embodiments, a tablet of the invention has a higher tensile strength, or a higher tabletability, as compared to a tablet prepared from a co-processed excipient comprising 75 wt% lactose and 25 wt% MCC (wherein the co-processed excipient is prepared by spray drying, in particular by a conventional spray drying process, i.e., where the spray drying is carried out with the slurry cooled down). See "circles" (25/75% MCC/Lactose, conventional process) in Figure 1.

In various embodiments, a tablet of the invention has a higher tensile strength, or a higher tabletability, as compared to a tablet prepared from a co-processed excipient comprising 75 wt% MCC and 25 wt% lactose, wherein the co-processed excipient is prepared by a conventional process, i.e., by a conventional spray drying process, where the spray drying is carried out with the slurry cooled down. See "triangles" (75/25% MCC/Lactose, conventional process) in Figure 1.

Additionally, or accordingly, in various embodiments, a co-processed excipient of the present invention provides for a tablet (of the invention) exhibiting a higher tensile strength, or a higher tabletability, as compared to a tablet prepared from a physical mixture (or physical admixture, "PAM") of the same concentration of lactose and MCC. See "squares" (75/25% MCC/Lactose, PAM (DC grades) in Figure 1.

Additionally, or accordingly, in various embodiments, a co-processed excipient of the present invention provides for a tablet (of the invention) exhibiting a higher tensile strength, or a higher tabletability, as compared to a tablet prepared from a co-processed excipient comprising 75 wt% lactose and 25 wt% MCC (wherein the co-processed excipient is prepared by spray drying, in particular by a conventional spray drying process, i.e., where the spray drying is carried out with the slurry cooled down). See "circles" (25/75% MCC/Lactose, conventional process) in Figure 1.

Additionally, or accordingly, in various embodiments, a co-processed excipient of the present invention provides for a tablet (of the invention) exhibiting a higher tensile strength, or a higher tabletability, as compared to a tablet prepared from a co-processed excipient comprising 75 wt% MCC and 25 wt% lactose, wherein the co-processed excipient is prepared by a conventional process, i.e., by a conventional spray drying process, where the spray drying is carried out with the slurry cooled down. See "triangles" (75/25% MCC/Lactose, conventional process) in Figure 1.

In the novel co-processed excipients of the present invention, the lactose content may comprise crystalline and amorphous forms of lactose. In various embodiments, it is preferred that the novel excipients of the present invention comprise lactose in crystalline and amorphous form. Accordingly, in the co-processed excipients of the present invention, the lactose is (or may be) a combination (or mixture) of crystalline and amorphous lactose.

Crystalline lactose can majorly exist in the form of α[alpha]-lactose and β[beta]-lactose. The α[alpha]-lactose typically is α[alpha]-lactose monohydrate. As described herein, it is preferred that the novel excipients of the present invention comprise lactose in crystalline and amorphous form, wherein the crystalline lactose comprises (or consists of) α[alpha]-lactose and/or (anhydrous) β[beta]-lactose. As further described herein, the combination (or mixture) of crystalline and amorphous lactose may comprise (or consist of) α[alpha]-lactose and/or (anhydrous) β[beta]-lactose, and amorphous lactose. The α[alpha]-lactose typically is α[alpha]-lactose monohydrate.

In various embodiments that may be considered preferred embodiments, the lactose is a combination (or mixture) of crystalline α[alpha]-lactose (monohydrate) and amorphous lactose, more specifically a combination (or mixture) consisting of crystalline α[alpha]-lactose (monohydrate) and amorphous lactose.

The current monographs for lactose described in the European Pharmacopeia (EP) or the United States Pharmacopeia - National Formulary (USP-NF) do not describe any mandatory testing for the apparent amorphous content. No reference to amorphous lactose can be found in any of these monographs, except for the USP-NF monograph on lactose monohydrate. This monograph includes only a note in the definition section to explain that lactose monohydrate *"may contain varying proportions of amorphous lactose".*

The embodiments pertaining to the lactose of the novel co-processed excipients apply likewise to the lactose used in the methods of preparing the novel co-processed excipients described herein, in particular as regards all aspects and embodiments described herein pertaining to preparing the aqueous suspension of MCC and lactose, wherein the dry mass of the aqueous suspension comprises MCC and lactose in a (mass) ratio (in the range) of about 60:40 to about 90:10, or comprises about 60-90 wt% MCC and about 10-40% lactose.

The novel excipient generated by the manufacturing process of the present invention is characterized by a specific combination of lactose and cellulose, in particular microcrystalline cellulose (MCC). More specifically, the novel excipient generated by the manufacturing process of the present invention is characterized by a specific combination of MCC and lactose in a (mass) ratio (in the range) of about 60:40 to about 90:10, or is characterized by a specific combination of about 60-90 wt% MCC and about 10-40 wt% lactose.

The cellulose is fine-particle or microcrystalline cellulose. In preferred embodiments of the present invention, in particular in preferred embodiments of the process of manufacture of the present invention, the MCC in the step of preparing an aqueous suspension of 60-90 wt% MCC has a (median) particle size of <100 µm, more specifically a median particle size of <100 µm.

Disclosed herein, but not forming part of the invention, is the use of powdered cellulose instead of MCC in the means and methods of the present disclosure. Accordingly, further disclosed herein, but not forming part of the invention, are all aspects and embodiments described herein using powdered cellulose instead of MCC.

Cellulose, in particular powdered cellulose, is an excipient that is used in the pharmaceutical industry. Likewise, microcrystalline cellulose (MCC) is known as an excipient that is used in the pharmaceutical industry. MCC is the same as cellulose, except that it meets USP standards. MCC may be considered as the (isolated) crystalline portion of cellulose fibers.

The present invention encompasses modified MCCs, e.g., silicified MCC, which is known as SMCC. The present invention further encompasses SCMC-MCC, which is MCC modified with sodium carboxy-methyl cellulose (SCMC).

The present invention encompasses different MCCs that may be characterized by, e.g., a different particle size distribution or different bulk density. As further described herein, MCCs may be mixed with other substances.

As described herein, particle sizes described herein can be determined by laser diffraction. As described elsewhere herein, the average (median) particle size can be determined by laser diffraction measurement (x50; wherein x50 represents the volume based median particles size, 50% of the particles above, 50% of the particle below).

The specific combination of lactose and MCC according to the present invention and their specific process of manufacture provide for the advantageous properties of the novel excipients of the present invention.

The production of the novel excipient of the present invention is carried out by co-processing via spray drying. For this purpose, MCC and lactose are provided according to the (mass) ratios / weight percentages described herein and dissolved or suspended in a suitable solvent. Preferably, the solvent is water. Specifically, the manufacturing process of the present invention is characterized by particular process steps, which include (i) preparing an aqueous suspension of MCC, wherein the dry mass of the aqueous suspension comprises about 60-90 wt% MCC; (ii) adding lactose to the aqueous suspension of (i), wherein the dry mass of the aqueous suspension thus obtained comprises about 60-90 wt% MCC and about 10-40 wt% lactose; (iii) heating the aqueous suspension of (ii) to a temperature of at least about 50°C to form a heated slurry; and (iv) spray-drying the heated slurry of (iii), thereby obtaining a co-processed excipient comprising about 60-90 wt% MCC and about 10-40 wt% lactose (or comprising MCC and lactose in a (mass) ratio (in the range) of about 60:40 to about 90:10).

As described herein, the aqueous suspension of MCC and lactose may be prepared in one step, i.e., MCC and lactose are stirred together in the solvent (water), which means that the lactose is not added after the aqueous suspension of MCC has been prepared. Furthermore, as described elsewhere herein, the preparation of the aqueous suspension of MCC and lactose may already contain a heating or warming of the suspension to a temperature of at least about 50°C, in which case there will be no separate heating step, i.e., the heated aqueous suspension (heated slurry) thus obtained can be spray dried directly.

As described herein, although the term "slurry" or "heated slurry" is clear to the one of ordinary skill in the art, the terms "heated slurry" and "heated aqueous suspension" may be used interchangeably, if deemed necessary or expedient.

In various embodiments of the present invention, the concentration of the aqueous suspension is a maximum of 40% (wt% in relation to the dry weight of the suspension). In preferred embodiments, the concentration of the aqueous suspension can be about 30%. Accordingly, in various embodiments of the present invention, the dry mass of the (heated) aqueous suspension is at most 40 wt%, preferably no more than 30 wt%. Preferably, the dry mass of the (heated) aqueous suspension is between about 20 wt% and about 30 wt%. Accordingly, in various embodiments of the present invention, the dry mass of the heated slurry aqueous suspension may be below about 20 wt%. Likewise, or accordingly, in various embodiments of the present invention, the dry mass of the heated slurry is at most 40 wt%, preferably no more than 30 wt%. Preferably, the dry mass of the heated slurry is between about 20 wt% and about 30 wt%. In various embodiments, the dry mass of the heated slurry may be below about 20 wt%.

As described herein, the terms "(heated) aqueous suspension" and "(heated) aqueous solution" may be used interchangeably, if deemed necessary or expedient.

It has surprisingly been found that the claimed process steps provide for a co-processed excipient that exhibits surprising properties in terms of tensile strength and/or solid fraction.

It has surprisingly been found that a tablet prepared from the novel excipient obtained by the novel manufacturing process according to the present invention has a higher tensile strength, or a higher tabletability (tensile strength vs. compression pressure) and a better compactibility (relationship between tensile strength and solid fraction (or porosity), as compared to a tablet prepared from a physical mixture of the same concentration of lactose and MCC, or as compared to a tablet prepared by conventional spray-drying of the same concentration of lactose and MCC. The results are shown in **Figures 1****,** **2** and **3****.** It has further surprisingly been found that a tablet prepared from the novel excipient obtained by the novel manufacturing process according to the present invention has a higher tensile strength, or a higher tabletability (tensile strength vs. compression pressure) and a better compactibility (relationship between tensile strength and solid fraction (or porosity), as compared to a tablet prepared from spray-dried MCC (that is also marketed for direct compression), as shown in **Figure 2** **and** **3****.** Figures 2 and 3 further show that a tablet according to the present invention has a higher tensile strength, or a higher tabletability (tensile strength vs. compression pressure) and a better compactibility (relationship between tensile strength and solid fraction or porosity), as compared to a tablet prepared from a co-processed excipient comprising 25 wt% MCC and 75 wt% lactose.

**Figures 8** **and** **9** demonstrate the improved/advantageous properties in terms of tensile strength, or tabletability (tensile strength vs. compression pressure) for tablets prepared from the novel excipients of the present invention and loaded with an active pharmaceutical ingredient, Paracetamol.

**Figure 3** shows the improved/advantageous properties of the novel excipients of the present invention in terms of a compactibility KPI comparison against tablets prepared from a physical mixture of the same concentration of lactose and MCC, or tablets prepared by conventional spray-drying of the same concentration of lactose and MCC. Figure 3 also shows the improved/advantageous properties of the novel excipients of the present invention in terms of a compactibility KPI comparison against tablets prepared from spray-dried MCC (used for DC). Figure 3 further shows the improved/advantageous properties of the novel excipients of the present invention in terms of a compactibility KPI comparison against tablets prepared from a co-processed excipient comprising 25 wt% MCC and 75 wt% lactose.

The claimed process of manufacture generates a co-processed excipient comprising 10-40 wt% lactose and 60-90 wt% MCC (or comprising MCC and lactose in a (mass) ratio (in the range) of about 60:40 to about 90:10).

In preferred embodiments, the co-processed excipient comprises 20-30 wt% lactose and 70-80 wt% MCC (or comprises MCC and lactose in a (mass) ratio (in the range) of about 70:30 to about 80:20). In preferred embodiments, the co-processed excipient comprises 25-40 wt% lactose and 60-75 wt% MCC (or comprises MCC and lactose in a (mass) ratio (in the range) of about 60:40 to about 75:25). In particularly preferred embodiments, the co-processed excipient of the present invention comprises about 75 wt% MCC, preferably about 75 wt% MCC and about 25 wt% lactose. In particularly preferred embodiments, the co-processed excipient of the present invention comprises about 60 wt% MCC, preferably about 60 wt% MCC and about 40 wt% lactose.

As described elsewhere herein, the co-processed excipient preferably comprises 25-40 wt% lactose and 60-75 wt% MCC, and can in particular comprise 30-40 wt% lactose and 60-70 wt% MCC. Accordingly, in particularly preferred embodiments, the co-processed excipient comprises 30-40 wt% lactose and 60-70 wt% MCC (or comprises MCC and lactose in a (mass) ratio (in the range) of about 60:40 to about 70:30). In a more particularly (or specifically) preferred embodiment, the co-processed excipient of the present invention comprises about 65 wt% MCC, more specifically about 65 wt% MCC and about 35 wt% lactose.

The present invention encompasses preferred embodiments, in which the co-processed excipient comprises 31-39 wt% lactose and 61-69 wt% MCC (or comprises MCC and lactose in a (mass) ratio (in the range) of about 61:39 to about 69:31). More preferably, the co-processed excipient comprises 32-38 wt% lactose and 62-68 wt% MCC (or comprises MCC and lactose in a (mass) ratio (in the range) of about 62:38 to about 68:32). Still more preferably, the co-processed excipient comprises 33-37 wt% lactose and 63-67 wt% MCC (or comprises MCC and lactose in a (mass) ratio (in the range) of about 63:37 to about 67:33). Even more preferably, the co-processed excipient comprises 34-36 wt% lactose and 64-66 wt% MCC (or comprises MCC and lactose in a (mass) ratio (in the range) of about 64:36 to about 66:34).

Also described herein and forming part of the invention are embodiments, wherein the co-processed excipient comprises 20-40 wt% lactose and 60-80 wt% MCC (or comprises MCC and lactose in a (mass) ratio (in the range) of about 60:40 to about 80:20). Also described herein and forming part of the invention are embodiments, wherein the co-processed excipient comprises 15-40 wt% lactose and 60-85 wt% MCC (or comprises MCC and lactose in a (mass) ratio (in the range) of about 60:40 to about 85:15).

Also described herein and forming part of the invention are embodiments, wherein the co-processed excipient comprises 10-25 wt% lactose and 75-90 wt% MCC (or comprises MCC and lactose in a (mass) ratio (in the range) of about 75:25 to about 90:10).

The percentage ranges described above apply accordingly to the methods of preparing a co-processed excipient of the invention described elsewhere herein. Accordingly, the present invention encompasses in particularly preferred embodiments that the method of preparing a co-processed excipient comprises preparing a co-processed excipient comprising 25-40 wt% lactose and 60-75% wt% MCC, wherein the method comprises: (i) preparing an aqueous suspension of MCC; (ii) adding lactose to the aqueous suspension of (i), wherein the dry mass of the aqueous suspension thus obtained comprises about 60-75 wt% MCC and about 25-40 wt% lactose; (iii) heating the aqueous suspension of (ii) to a temperature of at least about 50°C to form a heated slurry; (iv) spray-drying the heated slurry of (iii) and thereby obtaining a co-processed excipient comprising 25-40 wt% lactose and 60-75 wt% MCC (or comprises MCC and lactose in a (mass) ratio (in the range) of about 60:40 to about 75:25).

As described herein, in other or further preferred embodiments of the process of manufacture, the step of preparing an aqueous suspension of MCC, wherein the dry mass of the aqueous suspension comprises 60-90 wt% MCC, comprises heating the aqueous solvent to a temperature in the range of 50°C to 100°C.

As described elsewhere herein, in preferred embodiments of the present invention, the concentration of the suspension (or solution) or the slurry is a maximum of 40% (wt% in relation to the dry weight of the slurry). In various embodiments, the concentration of the suspension (or solution) or slurry is no more than about 30%, preferably in the range between 20-30%.

As described herein, the terms "co-processed excipient", "pharmaceutical excipient", or simply "excipient" may be used interchangeably herein, if deemed necessary or expedient.

It has surprisingly been found that spray drying produces spherical particles of the novel co-processed excipient, with a highly reproducible particle size and particle size distribution and a special micro-porous structure. **Figure 6** illustrates the specific particle structure of the novel excipients provided by the present invention. It has surprisingly been found that MCC/Lactose compositions according to the present invention exhibit advantageous properties in terms of porosity, which is beneficial for compressibility and (increasing) drug load of tablets, in particular as regards high dose tablets. In this regard, **Figure 7** shows an increased normalized volume (pore volume) for MCC/lactose compositions of the invention, in particular for MCC/lactose compositions of the invention that are prepared according to the process of the present invention.

The novel excipient of the present invention can be used to produce tablets that have a high breaking strength even at low pressures.

Surprisingly, tablets generated from the novel excipient according to the present invention by direct tableting have a greater breaking strength at a given compression pressure than tablets produced from the physical mixture of the components of the co-processed excipient, and are also considered to have a greater breaking strength at a given compression pressure as compared to granulates produced by wet granulation or by a spray drying process.

Accordingly, the novel co-processed excipient of the present invention shows properties that meet requirements that are necessary in particular for tableting excipient used for direct tableting. As shown herein, the novel co-processed excipient of the present invention exhibits good tabletability and compressibility with low compression pressure, as well as a good flowability. It has been demonstrated that a composition according to the invention typically exhibits an Angle of Repose between 31 and 35°. Accordingly, the novel co-processed excipient of the present invention can be characterized by an angle of repose between 31 and 35°.

The novel co-processed excipient allows producing tablets with a high breaking strength (tensile strength), as shown in Examples 1 and 2 and the corresponding Figures 1 and 2. In Figure 1, the measured breaking force (calculated tensile strength) of the exemplary co-processed excipient of the invention and tensile strengths of compacts made from granulates and physical mixtures consisting of the same composition components against the respective compression pressure It is clear to see that the novel co-processed excipients have higher tensile strengths at the same compression pressure, especially in the range of the preferred average pressing pressure of 150 to 250 MPa.

In preferred embodiments of the present invention, the median diameter of the particles of the co-processed excipient is in the range of from about 60 µm to about 150 µm.

A preferred solvent for the manufacturing process is water. If water is used as solvent, the aqueous suspension comprising 10-40 wt% lactose and 60-90 wt% MCC may be heated to a temperature between 50°C and 100°C, to form a heated slurry comprising 10-40 wt% lactose and 60-90 wt% MCC. In a preferred embodiment, water is used as the solvent and the aqueous suspension is heated to a temperature of 70°C to 80°C.

Subsequently, the heated slurry is directly spray-dried, *i.e.,* there is no cooling (down) of the slurry prior to spray-drying. The spray-drying of the present invention is preferably performed at a temperature in the range of about 140°C to about 225°C. A preferred temperature range is about 145°C to about 200°C, more preferably about 150°C to about 180°C. In other preferred embodiments, the heated slurry is sprayed at a temperature of about 190°C to about 200°C. In other preferred embodiments, the heated slurry is sprayed at a temperature of about 180°C to about 190°C.

The pressure in the environment into which the droplets are introduced is in the range of about 0 to about 2.0 bar, preferably from about 0.003 to about 1.5 bar, particularly preferably from about 0.005 to about 1.0 bar.

Suitable atomiser nozzles are, for example, single-substance, dual-substance or multi-substance pressure nozzles, such as turbulence, flat jet, impingement or hollow cone pressure nozzles, pneumatic nozzles, but also ultrasonic nozzles.

Single-substance nozzles may be operated at a nozzle pressure of 20 to 250 bar, preferably 30 to 200 bar, and two-substance or multi-substance nozzles at a nozzle pressure of 0.1 to 10 bar, preferably 0.3 to 5 bar.

Atomizing a liquid and/or suspension in an environment with increased temperature and possibly reduced pressure results in the liquid being at least partially removed from the droplets. This process is known in the art as spray drying.

After atomisation of the heated slurry, (residual) moisture may further be removed from the obtained product under ambient conditions until the content of free moisture in the particles of the new excipient is < 8 wt.%, preferably < 6 wt.%, particularly preferably < 5wt.% relative to the total mass of the particles. The total water content (according to Karl Fischer) may be in the range between 2-5%. Accordingly, in various embodiments, the manufacturing process may comprise a further step of post-drying, in particular a further step of post-drying in a fluidized bed. Such a further post-drying may be carried out at a temperature in the range of 30°C to 60°C.

The average (median) particle size resulting from the manufacturing process of the present invention can be in the range of 50-200 µm. The average (median) particle size can be determined by laser diffraction measurement (x50; wherein x50 represents the volume based median particles size, 50% of the particles above, 50% of the particle below). More specifically, the average (median) particle size resulting from the manufacturing process of the present invention can be in the range of 60-180 µm. In various embodiments, the average (median) particle size resulting from the manufacturing process of the present invention can be in the range of 60-160 µm, or in the range of 60-150 µm, or even in the range of 80-150 µm. Preferred particle sizes are in the range of 70-90 µm. In various embodiments, the preferred particle sizes are in the range of 70-80 µm. In various other embodiments, the preferred particle sizes are in the range of 80-90 µm.

Disclosed herein, but not forming part of the invention, is a (pharmaceutical) blend comprising an active ingredient, in particular an active pharmaceutical ingredient (API), and the novel co-processed excipient of the present invention. Further disclosed herein, but not forming part of the invention, is a (pharmaceutical) blend comprising an active substance that may be a natural product and the novel co-processed excipient of the present invention. The terms "blend" and "(blend) formulation" may be used interchangeably herein.

The blend may be a powder blend, wherein the powder blend may be a wettable powder that forms a suspension when mixed with water prior to administration.

Water activity, or Aw, is the isothermal ratio of a vapor pressure of a product, as opposed to pure water. A powder (blend/formulation) or dry powder (blend/formulation) as described herein may have a water activity (Aw) typical for the most common pharmaceutical dosage form, compressed tablets, i.e., in the range of 0.3 to 0.4. Likewise, a powder blend as described herein may have a water activity (Aw) typical for the most common pharmaceutical dosage form, compressed tablets, i.e., in the range of 0.3 to 0.4. Further, a (pharmaceutical) blend or formulation composition as described herein may also have a water activity (Aw) in the range of 0.3 to 0.4.

As described herein, the active (pharmaceutical) ingredient or active substance may be considered to be dispersed in, or encapsulated by, the novel co-processed excipient of the present invention.

A co-processed excipient of the present invention may optionally comprise further excipients or additives. Likewise, a blend or formulation disclosed herein, may optionally comprise further excipients or additives.

Conventional excipients and additives include, but are not limited to, disintegrants, flavoring agents, stabilizers, fillers, coloring agents, lubricants and the like. Suitable disintegrants are, e.g. sodium croscarmellose, crospovidone sodium starch glycolate. Suitable lubricants or glidants are, e.g., magnesium stearate, stearic acid, sodium stearyl fumarate, or colloidal silicon dioxide.

As described herein, "wt%" refers to, or preferably refers to, the dry mass (more specifically the total dry mass), in particular the (total) dry mass of the composition or excipient. In this regard, the composition preferably means the composition of the co-processed excipient of the present invention, which comprises a (co-processed) combination of 10-40 wt% lactose and 60-90 wt% microcrystalline cellulose (MCC), or a (co-processed) combination of MCC and lactose in a (mass) ratio (in the range) of about 60:40 to about 90:10.

As further described herein, the term "dry mass" in relation to components of the co-processed excipient of the present invention typically denominates the dry mass including water content, if any, in its compendial limits for water in regard to the respective components of the composition. The water content means water that may be present as a hydrate, e.g. in crystal form, as well as in an adsorbed form on the surface of a given component. Accordingly, and as will be appreciated by a person of ordinary skill in the art, the term "dry mass" in relation to, e.g., (alpha) lactose monohydrate, typically denominates the dry mass of the lactose including the hydrate water, and may further include surface water, if any. These considerations apply likewise also to the cellulose (MCC) component of the co-processed excipient of the present invention.

The co-processed excipient of the present invention encompasses hydrated components as well as anhydrous forms of the components, lactose and MCC.

The terms "dry mass" and "dry weight" or "dry matter" may be used interchangeably herein.

The present invention encompasses the novel co-processed excipients of the present invention in powder form or granule form. In particular, the present invention provides the novel co-processed excipients as a powder or as granules.

The present invention provides the use of an aqueous suspension for generating or preparing a co-processed excipient, wherein the dry mass of the aqueous suspension comprises about 10-40 wt% lactose and about 60-90 wt% microcrystalline cellulose (MCC), wherein the lactose is dissolved in the liquid phase of the aqueous suspension of MCC, in accordance with aspects and embodiments of the invention described elsewhere herein. Additionally, or alternatively, the present invention provides the use of an aqueous suspension for generating or preparing a co-processed excipient, wherein the aqueous suspension comprises microcrystalline cellulose (MCC) and lactose in a (mass) ratio (in the range) of about 60:40 to about 90:10, wherein the lactose is dissolved in the liquid phase of the aqueous suspension of MCC, in accordance with aspects and embodiments of the invention described elsewhere herein. All aspects and embodiments described elsewhere herein pertaining to preferred embodiments concerning the mass ratio of MCC and lactose, or the wt% of lactose and MCC, apply likewise to the use of the aqueous suspension for generating or preparing a co-processed excipient.

As will be appreciated by a person of ordinary skill in the art, a composition described herein by wt% (in particular the novel co-processed excipients described herein) cannot have a weight % (wt%) greater than 100. Accordingly, a person of ordinary skill in the art will appreciate that aspects and embodiments of the compositions of the invention (in particular of the novel co-processed excipients of the invention) as described herein are considered to describe compositions (in particular co-processed excipients) having a weight % (wt%) not greater than 100.

As described elsewhere herein, the present invention can also be performed with steps (i) and (ii) of the methods described under items [1] and [8] (corresponding to claims 1 and 8) carried out in reverse order: (i) preparing an aqueous suspension of lactose; (ii) adding MCC to the aqueous suspension of (i) (wherein the dry mass of the aqueous suspension thus obtained comprises 60-90 wt% MCC and 10-40 wt% lactose). Accordingly, the present invention encompasses the following [1] to [13]. In this regard, and for the sake of completeness, all definitions and embodiments described elsewhere herein apply, *mutatis mutandis,* to the following [1] to [13].
[1] A co-processed excipient comprising 10-40 wt% lactose and 60-90 wt% microcrystalline cellulose (MCC), wherein the co-processed excipient is generated by a method comprising:
   (i) preparing an aqueous suspension of lactose;
   (ii) adding MCC to the aqueous suspension of (i), wherein the dry mass of the aqueous suspension thus obtained comprises 60-90 wt% MCC and 10-40 wt% lactose;
   (iii) heating the aqueous suspension of (ii) to a temperature of at least about 50°C to form a heated slurry;
   (iv) spray-drying the heated slurry of (iii);
   (v) obtaining a co-processed excipient comprising 10-40 wt% lactose and 60-90 wt% MCC.
[2] The co-processed excipient of [1], comprising 25-40 wt% lactose and 60-75 wt% MCC, or comprising 20-30 wt% lactose and 70-80 wt% MCC, or comprising 30-40 wt% lactose and 60-70 wt% MCC.
[3] The co-processed excipient of [1] or [2], comprising about 60 wt% MCC, more specifically comprising about 60 wt% MCC and about 40 wt% lactose. In various preferred embodiments, the co-processed excipient comprises about 65 wt% MCC, more specifically about 65 wt% MCC and about 35 wt% lactose. In various other preferred embodiments, the co-processed excipient comprises about 75 wt% MCC, more specifically about 75 wt% MCC and about 25 wt% lactose.
   Accordingly, a preferred range is at least about 60% wt% MCC to at least about 75 wt% MCC, and/or at least about 40 wt% lactose to at least about 25 wt% lactose.
[4] The co-processed excipient of any one of [1] to [3], wherein the median diameter of the particles of the excipient is in the range from about 60 µm to about 150 µm.
[5] A tablet prepared from the co-processed excipient according to any one of [1] to [4], preferably by direct compression.
[6] The tablet of [5], which has a higher tensile strength, or a higher tabletability (tensile strength vs. compression pressure), as compared to a tablet prepared from a physical mixture of the same concentration of lactose and MCC. The terms "physical mixture" and "physical admixture" may be used interchangeably herein. As further described herein, the tablet of [6] has a higher tensile strength, or a higher tabletability (tensile strength vs. compression pressure), as compared to a direct compression (DC) product of the individual components prepared by spray drying of lactose, in particular (alpha) lactose monohydrate, or MCC, respectively. A known spray-dried alpha lactose monohydrate DC product is marketed as FlowLac^{®}. As can be derived from Figure 1, a tablet prepared from a co-processed excipient of the present invention, in particular from a co-processed excipient comprising 25-40 wt% lactose and 60-75 wt% microcrystalline cellulose (MCC), shows advantages properties in tensile strength, in particular a tensile strength of at least 5.0 (MPa), or greater than or ≥ 5.0 (MPa), at a compression pressure of 200 MPa. As further evidenced by Figure 2, the tensile strength at a compression pressure of 200 MPa can be in the range between 5.0 MPa and 6.5 MPa. In various embodiments, the tensile strength at a compression pressure of 200 MPa is in the range of (about) 5.4 MPa and (about) 6.1 MPa, more specifically in the range of (about) 5.5 MPa and (about) 6.0 MPa. As described elsewhere herein, in particularly preferred embodiments, a co-processed excipient comprises 30-40 wt% lactose and 60-70 wt% MCC. While the use of the term "compression pressure" follows the United States Pharmacopeia (United States Pharmacopeia (2023). General Chapter, 〈1062〉 Tablet Compression Characterization. USP-NF. Rockville, MD: United States Pharmacopeia. DOI: https://doi.org/10.31003/USPNF_M99395_02_01. Doc ID: GUID-A53EEC6D-BF32-464C-88BC-5303A5628C2C_2_en-US), the terms "compression pressure" and "compaction pressure" may be used interchangeably in the art. Accordingly, the terms "compression pressure" and "compaction pressure" may also be used interchangeably in the present invention.
[7] The tablet of [5], which has a higher tensile strength, or a higher tabletability (tensile strength vs. compression pressure), as compared to a tablet prepared from a co-processed excipient comprising 75 wt% lactose, in particular 75 wt% (alpha) lactose monohydrate and 25 wt% MCC.
[8] A method of preparing a co-processed excipient comprising 10-40 wt% lactose and 60-90 wt% microcrystalline cellulose (MCC), wherein the method comprises:
   (i) preparing an aqueous suspension of lactose;
   (ii) adding MCC to the aqueous suspension of (i), wherein the dry mass of the aqueous suspension thus obtained comprises 60-90 wt% MCC and 10-40 wt% lactose;
   (iii) heating the aqueous suspension of (ii) to a temperature of at least about 50°C to form a heated slurry;
   (iv) spray-drying the heated slurry of (iii);
   (v) obtaining a co-processed excipient comprising 10-40 wt% lactose and 60-90 wt% MCC.
[9] The method of [8], wherein:
   a) the MCC of step (i) has a (median) particle size of <100 µm; and/or
   b) step (iii) comprises heating the aqueous suspension to a temperature in the range from about 50°C to about 100°C.
[10] The method of [8] or [9], wherein the spray-drying of step (iv) is performed at a temperature in the range of about 140°C to about 225°C.
[11] Use of the co-processed excipient according to any one of [1] to [4] in a process of preparing a tablet by direct compression.
[12] Use of an aqueous suspension for preparing a co-processed excipient comprising 10-40 wt% lactose and 60-90 wt% microcrystalline cellulose (MCC), wherein the dry mass of the suspension comprises 10-40 wt% lactose and 60-90 wt% MCC, and wherein the lactose is dissolved in the liquid phase of an aqueous suspension of MCC, and wherein the co-processed excipient is generated by a method comprising:
   (i) heating the aqueous suspension to a temperature of at least about 50°C to form a heated slurry;
   (ii) spray-drying the heated slurry of (i);
   (iii) obtaining a co-processed excipient comprising 10-40 wt% lactose and 60-90 wt% MCC.
   The step of heating the aqueous suspension is preferably performed at a temperature in the range from about 50°C to about 100°C.
[13] A method of preparing a tablet comprising direct compression of a (pharmaceutical) blend of an active pharmaceutical ingredient and the co-processed excipient of any one of [1] to [4].

### EXAMPLES

The examples verify and confirm the advantageous properties of the novel co-processed excipients of the present invention.

### Methods for tablet compression characterization (tabletability, compactibility and compressibility) applied herein include the following:

To characterize tablet compression, a force test following the recommendations of the United States Pharmacopeia (<1062>¹ Tablet Compression Characterization) has been established. Tablets are compressed with five (5) different compression pressures in the range from approx. 20 to 300 MPa using an instrumented tablet press (Styl'One). IPC tests are performed on 10 resulting tablets (tablet weight, diameter and breaking force/crushing strength).
¹ United States Pharmacopeia (2023). General Chapter, 〈1062〉 Tablet Compression Characterization. USP-NF. Rockville, MD: United States Pharmacopeia. DOI: https://doi.org/10.31003/USPNF_M99395_02_01. Doc ID: GUID-A53EEC6D-BF32-464C-88BC-5303A5628C2C_2_ en-US.

In order to have a more tablet size/ format independent comparison, tensile strength and compression pressure are used (USP <1217>² Tablet Breaking Force, <1062> Tablet Compression Characterization). $\begin{matrix}Compression Pressure & CP = 4 ⋅ \frac{{F}_{Compression}}{π ⋅ {D}^{2}} ⋅ 1000 & \left[kN*1000/{mm}^{2}=MPa\right] \\ Tensile Strength & TS = 2 ⋅ \frac{{BF}_{Tablets}}{π ⋅ D ⋅ h} & \left[N/{mm}^{2}=MPa\right] \left(cylindrical tablet\right) \\ tablet Density & {ρ}_{Tab} = 4 ⋅ \frac{{m}_{Tab}}{π ⋅ {D}^{2} ⋅ h} & \left[mg/{mm}^{3}=g/{cm}^{3}\right] \\ Solid Fraction & SF = \frac{{ρ}_{Tab}}{{ρ}_{Material}} & \left[-\right] \left(Porosity=1-Solid Fraction\right)\end{matrix}$ Equation 1: Compression Pressure, Tensile Strenght, Tablet Density and Solid Fraction
*F_{Compression}* = Compression Force in kN
*BF_{Tablets}* = Breaking Force / Crushing Strength (avg. IPC) in N
D = Tablet Diameter (avg. IPC) in mm
h = Tablet Thickness (avg. IPC) in mm
*ρ_{Material}* = True Density (Material)
² United States Pharmacopeia (2024). General Chapter, 〈1217〉 Tablet Breaking Force. USP-NF. Rockville, MD: United States Pharmacopeia. DOI: https://doi.org/10.31003/USPNF_M99937_02_01; Doc ID: GUID-4DC4CB4A-5FB0-4F87-8F72-45582BEAC6E9_2_en-US.

The tensile strength at a compaction pressure of 200 MPa ("TS@CP200"; Fig. 2) is calculated using linear interpolation from the tabletability profile (tensile strength vs compaction pressure). The tensile strength at a solid fraction of 0.85 ("TS@SF85"; Fig. 3) is calculated using interpolation based on exponential fit from the compactibility profile (tensile strength vs compaction pressure).

### Angle of Repose

The angle of repose is a parameter in determining the flowability and storage properties of powders and granules. The method according to ISO 4324 provides a standardized method for measuring the angle of repose, ensuring consistency and reliability in the assessment of these materials. Based on the European Pharmacopeia as well as the United States Pharmacopeia (Ph.Eur. 2.9.36 / USP <1174>) material can be classified to have "good" flow properties if the Angle of Repose is between 31-35°.

| Angle of Repose [°] | Flow Property |
|---|---|
| 25-30 | Excellent |
| **31-35** | **Good** |
| 36-40 | Fair (aid not needed) |
| 41-45 | Passable (may hang up) |
| 46-55 | Poor (must agitate, vibrate) |
| 56-65 | Very poor |
| > 66 | Very, very poor |

**Particle Size:** Determination by laser diffraction (dry dispersion method with Sympatec R5, 0.5 bar dispersion pressure), x50 represents the volume based **median** particles size (50% of the particles above, 50% of the particle below).

**Water Content:** Karl Fischer Method.

**Loss on Drying:** Oven method (80°C, 2 hours drying).

### Example 1: Co-processed excipient made from 40% lactose and 60% MCC

| | |
|---|---|
| Basic composition: | 40% lactose, median particle size 10-50 µm (laser diffraction) |
| | 60% MCC, median particle size 45-80 µm (laser diffraction) |
| Additives | None |
| Solvent | 100% Water |
| Dry Mass | 22% |

13.2 kg of microcrystalline cellulose and 8.8 kg of lactose are stirred into 78 kg of water in the preparation tank. The batch is then heated to 75°C to 80°C. Specifically, or alternatively, a first aqueous suspension of 13.2 kg MCC is prepared, and 8.8 kg lactose are then added to the aqueous suspension of 13.2 kg MCC. The heated slurry is subsequently sprayed at 190-200°C in a spray tower. A dry powder with an average (median) particle size of 80-90 µm is obtained. This powder is then pressed into tablets on a tablet press at different pressing forces.

### Parameters:

| **Compression Pressure (MPa)** | **(Breaking) Tensile Strength (MPa)** |
|---|---|
| 75 | 2.0 |
| 127 | 3.8 |
| 181 | 5.5 |
| 234 | 6.8 |
| 289 | 7.3 |

Compression pressure to achieve a breaking strength of 2.0 MPa is only 62 MPa and solid fraction is only 0.69 (remaining porosity 0.31). Tensile strength reached at a solid fraction of 0.85 is 5.0 MPa. Tensile strength reached at a compression pressure of 200 MPa is 5.5 MPa.

### Example 2: Co-processed excipient made from 25% lactose and 75% MCC

| | |
|---|---|
| Basic composition: | 25% lactose, median particle size 10-50 µm (laser diffraction) |
| | 75% MCC, with 90% having a median particle size 45-80 µm (laser diffraction), and 10% with a maximum of 8% particles > 32 µm (air jet sieving) |
| Additives | None |
| Solvent | 100% Water |
| Dry Mass | 22% |

23.1 kg of microcrystalline cellulose and 7.7 kg of lactose are stirred into 109.2 kg of water in the preparation tank. The batch is then heated to 75°C to 80°C. Specifically, or alternatively, a first aqueous suspension of 23.1 kg MCC is prepared, and 7.7 kg lactose are then added to the aqueous suspension of 23.1 kg MCC. The heated slurry is subsequently sprayed at 180-190°C in a spray tower. A dry powder with an average (median) particle size of 70-80 µm is obtained. This powder is then pressed into tablets on a tablet press at different pressing forces.

### Parameters:

| **Compression Pressure (MPa)** | **(Breaking) Tensile Strength (MPa)** |
|---|---|
| 72 | 2.0 |
| 120 | 3.1 |
| 168 | 5.1 |
| 216 | 6.1 |
| 266 | 7.1 |

Compression pressure to achieve a breaking strength of 2.0 MPa is only 72 MPa and a solid fraction of only 0.625 (remaining porosity 0.375). Tensile strength reached at a solid fraction of 0.85 is 5.5 MPa. Tensile strength reached at a compression pressure of 200 MPa is 5.6 MPa.

In Figure 1, the measured breaking strengths of the examples according to the invention, and the measured breaking strengths of compacts made from granulates and physical mixtures consisting of the same composition components, are plotted against the respective compressing pressure. It is clear to see that the examples according to the invention have higher breaking strengths at the same compressing pressing pressure, especially in the range of the preferred average pressing pressure of 150 to 250 MPa.

Figure 6 illustrates the specific particle structure of the novel excipients provided by the present invention.

### Example 3: Mercury Porosimetry

Method: Quantachrome Poremaster (bulk sample volume 1 cm³, measurement range approx. 1000 µm-0.0036 µm pore diameter) - ISO 15901-1
The granular density includes contributions to particle volume from open, small pores. A common measurement method for this is mercury porosimetry (intrusion of mercury).

Such a pore size measurement based on mercury porosimetry is made using compositions of "100% lactose" (DC product of 100 wt % (alpha) lactose monohydrate (FlowLac^{®}); "25% MCC/75% lactose - conventional process" (i.e., a composition of 25/75 wt% MCC/Lactose prepared from a co-spray dried mixture of a composition of 25 wt% MCC and 75 wt% lactose; "conventional" spray-drying process); "60% MCC/40% lactose - conventional process" (i.e., a composition of 60/40 wt% MCC/Lactose prepared from a co-spray dried mixture of a composition of 60 wt% MCC and 40 wt% lactose; "conventional" spray-drying process), and "60% MCC/40% lactose - process according to the invention" (i.e., a composition of 60/40 wt% MCC/Lactose prepared from spray drying according to the present invention).

The results are shown in Figure 7.

### Example 4: Tabletability profile with 20% Paracetamol

A tabletability profile has been established using Paracetamol drug load. In particular, 20% Paracetamol (very fine crystals, with a median diameter of 26 µm), a DC excipient, or product and 1% silicon dioxide are blended for 10 minutes (Turbula blender, 72 rpm). Then addition of 1% magnesium stearate and blending for 2 min (Turbula blender, 72 rpm).

| Ingredients | **Formulation 1** | **Formulation 2** | **Formulation 3** | **Composition %** |
|---|---|---|---|---|
| Paracetamol, very fine | 187.5 mg | 187.5 mg | 187.5 mg | 20% |
| 25/75% MCC/ Lactose (conventional process) | 547.5 mg | -- | -- | 78% |
| 75/25% MCC/ Lactose, according example 2 | -- | 547.5 mg | -- | |
| Lactose, spray dried (DC grade) | -- | -- | 146.25 mg | |
| MCC (DC grade) | -- | -- | 438.75 mg | |
| Colloidal Silicon Dioxide | 7.5 mg | 7.5 mg | 7.5 mg | 1% |
| Magnesium Stearate | 7.5 mg | 7.5 mg | 7.5 mg | 1% |
| Sum | 750 mg | 750 mg | 750 mg | |

Tablet compression was performed using Korsch XL 100 rotary tablet press with round, biplane punches of 11 mm diameter, tablet weight 500 mg.

Parameters "tablet weight", "thickness", "diameter" and "breaking force" have been tested at a representative sample size for each compression force using an Erweka TBH 425 tablet hardness and combination tester.

Based on measured compression force and tablet breaking force the compression pressure and tensile strength have been calculated (reference USP <1217> Tablet Breaking Force, <1062> Tablet Compression Characterization).

The results are shown in Figure 8.

### Example 5: Tabletability profile with 50% Paracetamol

A tabletability profile has been established using 50% Paracetamol drug load. In particular Paracetamol (coarse; median diameter of 397 µm), a DC excipient, and 1% silicon dioxide are blended for 10 minutes (Turbula blender, 72 rpm).

Then addition of 1% magnesium stearate and blending for 2 min (Turbula blender, 72 rpm).

| **Ingredients** | **Formulation 1** | **Formulation 2** | **Composition %** |
|---|---|---|---|
| Paracetamol, coarse | 375 mg | 375 mg | 50% |
| 25/75% MCC/ Lactose (conventional process) | 360 mg | -- | 48% |
| 75/25% MCC/ Lactose, according example 2 | -- | 360 mg | |
| Colloidal Silicon Dioxide | 7.5 mg | 7.5 mg | 1% |
| Magnesium Stearate | 7.5 mg | 7.5 mg | 1% |
| Sum | 750 mg | 750 mg | |

Tablet compression was performed using Korsch XL 100 rotary tablet press with round, biplane punches of 11 mm diameter, tablet weight 500 mg.

Parameters "tablet weight", "thickness", "diameter" and "breaking force" have been tested at a representative sample size for each compression force using an Erweka TBH 425 tablet hardness and combination tester.

Based on measured compression force and tablet breaking force the compression pressure and tensile strength have been calculated (reference USP <1217> Tablet Breaking Force, <1062> Tablet Compression Characterization>).

The results are shown in Figure 9.

### Example 6: Co-processed excipient made from 35% lactose and 65% MCC

A co-processed excipient made from 35% lactose and 65% MCC is prepared in analogy to the co-processed excipients prepared in Examples 1 and 2.

| | |
|---|---|
| Basic composition: | 35% lactose, median particle size 10-50 µm (laser diffraction) |
| | 65% MCC, with 90% having a median particle size 45-80 µm (laser diffraction), and 10% with a maximum of 8% particles > 32 µm (air jet sieving) |
| Additives | None |
| Solvent | 100% Water |
| Dry Mass | 25% |

A batch prepared from microcrystalline cellulose and lactose (stirred into water in a preparation tank) is heated to 75°C to 80°C. Specifically, or alternatively, a first aqueous solution of lactose is prepared, and MCC is then added to the aqueous solution of lactose. The heated slurry is subsequently sprayed at 180-200°C in a spray tower. A dry powder with a median particle size of 60-90 µm is obtained. This powder is then pressed into tablets on a tablet press at different pressing forces.

### Parameters:

| **Compression Pressure (MPa)** | **(Breaking) Tensile Strength (MPa)** |
|---|---|
| 23 | 0.2 |
| 66 | 1.3 |
| 113 | 3.1 |
| 161 | 4.9 |
| 210 | 6.3 |

Compression pressure to achieve a breaking strength of 2.0 MPa is only 80 MPa and a solid fraction of only 0.75 (remaining porosity 0.25). Tensile strength reached at a solid fraction of 0.85 is 5.3 MPa. Tensile strength reached at a compression pressure of 200 MPa is 6.0 MPa.

The results are reflected in Figure 1 and Figure 2. As mentioned in Example 2 it is clear to see that, in comparison to the compacts made from granulates and physical mixtures consisting of the same composition components, the examples according to the invention have higher breaking strengths at the same compression pressure, especially in the range of the preferred average pressing pressure of 150 to 250 MPa.

## Claims

1. A co-processed excipient comprising 10-40 wt% lactose and 60-90 wt% microcrystalline cellulose (MCC), wherein the co-processed excipient is generated by a method comprising:
(i) preparing an aqueous suspension of MCC;
(ii) adding lactose to the aqueous suspension of (i), wherein the dry mass of the aqueous suspension thus obtained comprises 60-90 wt% MCC and 10-40 wt% lactose;
(iii) heating the aqueous suspension of (ii) to a temperature of at least about 50°C to form a heated slurry;
(iv) spray-drying the heated slurry of (iii);
(v) obtaining a co-processed excipient comprising 10-40 wt% lactose and 60-90 wt% MCC;
wherein a tablet prepared from the co-expressed excipient exhibits a tensile strength of ≥ 5.0 MPa at a solid fraction of 0.85.

2. The co-processed excipient of claim 1, comprising 25-40 wt% lactose and 60-75 wt% MCC, preferably comprising 25-35 wt% lactose and 65-75 wt% MCC.

3. The co-processed excipient of claim 1 or 2, comprising:
60 wt% MCC, in particular 60 wt% MCC and 40 wt% lactose; or
65 wt% MCC, in particular 65 wt% MCC and 35 wt% lactose; or
75 wt% MCC, in particular 75 wt% MCC and 25 wt% lactose.

4. The co-processed excipient of any one of claims 1 to 3, wherein the median diameter of the particles of the excipient is in the range from about 60 µm to about 150 µm, as determined by laser diffraction.

5. A tablet prepared from the co-processed excipient according to any one of claims 1 to 4, preferably by direct compression.

6. The tablet of claim 5, which has a higher tensile strength, or a higher tabletability, as compared to a tablet prepared from a physical mixture of the same concentration of lactose and MCC.

7. The tablet of claim 5, which has a higher tensile strength, or a higher tabletability, as compared to a tablet prepared from a co-processed excipient comprising 75 wt% lactose and 25 wt% MCC.

8. A method of preparing a co-processed excipient comprising 10-40 wt% lactose and 60-90% wt% microcrystalline cellulose (MCC), wherein the method comprises:
(i) preparing an aqueous suspension of MCC;
(ii) adding lactose to the aqueous suspension of (i), wherein the dry mass of the aqueous suspension thus obtained comprises 60-90 wt% MCC and 10-40 wt% lactose;
(iii) heating the aqueous suspension of (ii) to a temperature of at least about 50°C to form a heated slurry;
(iv) spray-drying the heated slurry of (iii);
(v) obtaining a co-processed excipient comprising 10-40 wt% lactose and 60-90 wt% MCC.

9. The method of claim 8, wherein:
a) the MCC of step (i) has a median particle size of <100 µm, as determined by laser diffraction; and/or
b) step (iii) comprises heating the aqueous suspension to a temperature in the range from about 50°C to about 100°C.

10. The method of claim 8 or 9, wherein the spray-drying of step (iv) is performed at a temperature in the range of about 140°C to about 225°C.

11. Use of the co-processed excipient according to any one of claims 1 to 4 in a process of preparing a tablet by direct compression.

12. Use of an aqueous suspension for preparing a co-processed excipient comprising 10-40 wt% lactose and 60-90 wt% microcrystalline cellulose (MCC),
wherein the dry mass of the suspension comprises 10-40 wt% lactose and 60-90 wt% MCC, and wherein the lactose is dissolved in the liquid phase of an aqueous suspension of MCC, and
wherein the co-processed excipient is generated by a method comprising:
(i) heating the aqueous suspension to a temperature of at least about 50°C to form a heated slurry;
(ii) spray-drying the heated slurry of (i);
(iii) obtaining a co-processed excipient comprising 10-40 wt% lactose and 60-90 wt% MCC.

13. A method of preparing a tablet comprising direct compression of a blend of an active pharmaceutical ingredient and the co-processed excipient of any one of claims 1 to 4.

## Patentansprüche

1. Co-prozessierter Hilfsstoff umfassend 10-40 Gew.-% Laktose und 60-90 Gew.-% mikrokristalline Cellulose (MCC), wobei der co-prozessierte Hilfsstoff durch ein Verfahren hergestellt wird, welches umfasst:
(i) Herstellen einer wässrigen Suspension von MCC;
(ii) Zugeben von Laktose zu der wässrigen Suspension aus (i), wobei die Trockenmasse der so erhaltenen wässrigen Suspension 60-90 Gew.-% MCC und 10-40 Gew.-% Laktose umfasst;
(iii) Erhitzen der wässrigen Suspension aus (ii) auf eine Temperatur von mindestens etwa 50 °C, um eine erhitzte Aufschlämmung zu bilden;
(iv) Sprühtrocknen der erhitzten Aufschlämmung aus (iii);
(v) Erhalten eines co-prozessierten Hilfsstoffs, welcher 10-40 Gew.-% Laktose und 60-90 Gew.-% MCC umfasst;
wobei eine aus dem co-prozessierten Hilfsstoff hergestellte Tablette bei einem Feststoffanteil von 0,85 eine Zugfestigkeit von ≥ 5,0 MPa aufweist.

2. Der co-prozessierte Hilfsstoff nach Anspruch 1, umfassend 25-40 Gew.-% Laktose und 60-75 Gew.-% MCC, vorzugsweise umfassend 25-35 Gew.-% Laktose und 65-75 Gew.-% MCC.

3. Der co-prozessierte Hilfsstoff nach Anspruch 1 oder 2, umfassend:
60 Gew.-% MCC, insbesondere 60 Gew.-% MCC und 40 Gew.-% Laktose; oder
65 Gew.-% MCC, insbesondere 65 Gew.-% MCC und 35 Gew.-% Laktose; oder
75 Gew.-% MCC, insbesondere 75 Gew.-% MCC und 25 Gew.-% Laktose.

4. Der co-prozessierte Hilfsstoff nach einem der Ansprüche 1 bis 3, wobei der Median-Durchmesser der Partikel des Hilfsstoffs im Bereich von etwa 60 µm bis etwa 150 µm liegt, bestimmt mittels Laserbeugung.

5. Tablette hergestellt aus dem co-prozessierten Hilfsstoff gemäß einem der Ansprüche 1 bis 4, vorzugsweise durch Direktverpressung.

6. Die Tablette nach Anspruch 5, die im Vergleich zu einer Tablette, die aus einer physikalischen Mischung aus Laktose und MCC in gleicher Konzentration hergestellt wurde, eine höhere Zugfestigkeit oder eine höhere Tablettierbarkeit aufweist.

7. Die Tablette nach Anspruch 5, die im Vergleich zu einer Tablette, die aus einem co-prozessierten Hilfsstoff umfassend 75 Gew.-% Laktose und 25 Gew.-% MCC hergestellt wurde, eine höhere Zugfestigkeit oder eine höhere Tablettierbarkeit aufweist.

8. Verfahren zur Herstellung eines co-prozessierten Hilfsstoffs umfassend 10-40 Gew.-% Laktose und 60-90 Gew.-% mikrokristalline Cellulose (MCC), wobei das Verfahren umfasst:
(i) Herstellen einer wässrigen Suspension von MCC;
(ii) Zugeben von Laktose zu der wässrigen Suspension aus (i), wobei die Trockenmasse der so erhaltenen wässrigen Suspension 60-90 Gew.-% MCC und 10-40 Gew.-% Laktose umfasst;
(iii) Erhitzen der wässrigen Suspension aus (ii) auf eine Temperatur von mindestens etwa 50 °C, um eine erhitzte Aufschlämmung zu bilden;
(iv) Sprühtrocknen der erhitzten Aufschlämmung aus (iii);
(v) Erhalten eines co-prozessierten Hilfsstoffs, welcher 10-40 Gew.-% Laktose und 60-90 Gew.-% MCC umfasst.

9. Verfahren nach Anspruch 8, wobei:
a) die MCC in Schritt (i) eine mediane Partikelgröße von <100 µm aufweist, bestimmt mittels Laserbeugung; und/oder
b) Schritt (iii) das Erhitzen der wässrigen Suspension auf eine Temperatur im Bereich von etwa 50 °C bis etwa 100 °C umfasst.

10. Verfahren nach Anspruch 8 oder 9, wobei die Sprühtrocknung in Schritt (iv) bei einer Temperatur im Bereich von etwa 140 °C bis etwa 225 °C durchgeführt wird.

11. Verwendung des co-prozessierten Hilfsstoffs gemäß einem der Ansprüche 1 bis 4 in einem Verfahren zur Herstellung einer Tablette durch Direktverpressung.

12. Verwendung einer wässrigen Suspension zur Herstellung eines co-prozessierten Hilfsstoffs, welcher 10-40 Gew.-% Laktose und 60-90 Gew.-% mikrokristalline Cellulose (MCC) umfasst,
wobei die Trockenmasse der Suspension 10-40 Gew.-% Laktose und 60-90 Gew.-% MCC umfasst und wobei die Laktose in der flüssigen Phase einer wässrigen Suspension von MCC gelöst ist, und
wobei der co-prozessierte Hilfsstoff durch ein Verfahren hergestellt wird, welches umfasst:
(i) Erhitzen der wässrigen Suspension auf eine Temperatur von mindestens etwa 50 °C, um eine erhitzte Aufschlämmung zu bilden;
(ii) Sprühtrocknen der erhitzten Aufschlämmung aus (i);
(iii) Erhalten eines co-prozessierten Hilfsstoffs, welcher 10-40 Gew.-% Laktose und 60-90 Gew.-% MCC umfasst.

13. Verfahren zur Herstellung einer Tablette umfassend das direkte Verpressen einer Mischung aus einem pharmazeutischen Wirkstoff und dem co-prozessierten Hilfsstoff gemäß einem der Ansprüche 1 bis 4.

## Revendications

1. Excipient co-traité comprenant 10 à 40 % en poids de lactose et 60 à 90 % en poids de cellulose microcristalline (MCC), dans lequel l'excipient co-traité est obtenu par un procédé comprenant:
(i) la préparation d'une suspension aqueuse de MCC;
(ii) l'ajout de lactose à la suspension aqueuse de l'étape (i), la masse sèche de la suspension aqueuse ainsi obtenue comprenant 60 à 90 % en poids de MCC et 10 à 40 % en poids de lactose;
(iii) chauffer la suspension aqueuse de (ii) à une température d'au moins environ 50 °C pour former une bouillie chauffée;
(iv) sécher par atomisation la bouillie chauffée de (iii);
(v) obtenir un excipient co-traité comprenant 10 à 40 % en poids de lactose et 60 à 90 % en poids de MCC;
dans lequel un comprimé préparé à partir de l'excipient co-traité présente une résistance à la traction ≥ 5,0 MPa pour une fraction solide de 0,85.

2. L'excipient co-traité selon la revendication 1, comprenant 25 à 40 % en poids de lactose et 60 à 75 % en poids de MCC, comprenant de préférence 25 à 35 % en poids de lactose et 65 à 75 % en poids de MCC.

3. L'excipient co-traité selon la revendication 1 ou 2, comprenant:
60 % en poids de MCC, en particulier 60 % en poids de MCC et 40 % en poids de lactose; ou
65 % en poids de MCC, en particulier 65 % en poids de MCC et 35 % en poids de lactose; ou
75 % en poids de MCC, en particulier 75 % en poids de MCC et 25 % en poids de lactose.

4. Excipient co-traité selon l'une quelconque des revendications 1 à 3, dans lequel le diamètre médian des particules de l'excipient est compris entre environ 60 µm et environ 150 µm, tel que déterminé par diffraction laser.

5. Comprimé préparé à partir de l'excipient co-traité selon l'une quelconque des revendications 1 à 4, de préférence par compression directe.

6. Comprimé selon la revendication 5, qui présente une résistance à la traction plus élevée ou une meilleure compressibilité par rapport à un comprimé préparé à partir d'un mélange physique de lactose et de MCC en concentrations égales.

7. Comprimé selon la revendication 5, qui présente une résistance à la traction plus élevée ou une meilleure compressibilité par rapport à un comprimé préparé à partir d'un excipient co-traité comprenant 75 % en poids de lactose et 25 % en poids de MCC.

8. Procédé de préparation d'un excipient co-traité comprenant 10 à 40 % en poids de lactose et 60 à 90 % en poids de cellulose microcristalline (MCC), dans lequel le procédé comprend:
(i) la préparation d'une suspension aqueuse de MCC;
(ii) l'ajout de lactose à la suspension aqueuse de l'étape (i), la masse sèche de la suspension aqueuse ainsi obtenue comprenant 60 à 90 % en poids de MCC et 10 à 40 % en poids de lactose;
(iii) chauffer la suspension aqueuse de (ii) à une température d'au moins environ 50 °C pour former une bouillie chauffée;
(iv) sécher par atomisation la bouillie chauffée de (iii);
(v) obtenir un excipient co-traité comprenant 10 à 40 % en poids de lactose et 60 à 90 % en poids de MCC.

9. Procédé selon la revendication 8, dans lequel:
a) le MCC de l'étape (i) présente une taille médiane de particules inférieure à 100 µm, telle que déterminée par diffraction laser; et/ou
b) l'étape (iii) comprend à chauffer la suspension aqueuse à une température comprise entre environ 50 °C et environ 100 °C.

10. Procédé selon la revendication 8 ou 9, dans lequel le séchage par atomisation de l'étape (iv) est effectué à une température comprise entre environ 140 °C et environ 225 °C.

11. Utilisation de l'excipient co-traité selon l'une quelconque des revendications 1 à 4 dans un procédé de préparation d'un comprimé par compression directe.

12. Utilisation d'une suspension aqueuse pour la préparation d'un excipient co-traité comprenant 10 à 40 % en poids de lactose et 60 à 90 % en poids de cellulose microcristalline (MCC),
dans laquelle la masse sèche de la suspension comprend 10 à 40 % en poids de lactose et 60 à 90 % en poids de MCC, et dans laquelle le lactose est dissous dans la phase liquide d'une suspension aqueuse de MCC, et
dans laquelle l'excipient co-traité est obtenu par un procédé comprenant:
(i) le chauffage de la suspension aqueuse à une température d'au moins environ 50 °C pour former une bouillie chauffée;
(ii) le séchage par atomisation de la bouillie chauffée de l'étape (i);
(iii) obtenir un excipient traité comprenant 10 à 40 % en poids de lactose et 60 à 90 % en poids de MCC.

13. Procédé de préparation d'un comprimé comprenant la compression directe d'un mélange d'un principe actif pharmaceutique et de l'excipient co-traité selon l'une quelconque des revendications 1 à 4.
